# EUROPEAN PATENT APPLICATION

(11) **EP 4 414 069 A1**
(43) Date of publication of application: **14.08.2024**
(21) Application number: 23155652.3
(22) Date of filing: 08.02.2023
(51) Int. Cl.: B01J 21/08, B01J 23/80, B01J 35/00, B01J 35/02, B01J 37/03, C07C 29/153

(54) **CORE-SHELL MATERIALS**

(71) Applicant: Oxford University Innovation Limited, Oxford, OX2 0JB (GB); SCG Chemicals Public Company Limited, 10800 Bangkok (TH)
(72) Inventor: O'HARE, Dermot, Oxford OX2 0JB (GB); CHEN, Chunping, Oxford OX2 0JB (GB); LYU, Meng, Oxford OX2 0JB (GB)
(74) Representative: HGF

(57) **Abstract**

New core-shell silica-LDH particles useful as starting materials in the formation of catalysts are described, as well as methods of preparing the core-shell particles. Also described are catalyst precursors and catalysts formed from the core-shell particles, the catalysts being suitable for catalysing the hydrogenation of CO₂ (or CO) to methanol.

## Description

### INTRODUCTION

The present invention relates to a particle (e.g. a plurality of particles, such as a powder) having a core-shell structure, as well as to catalyst precursors and catalysts formed therefrom. The present invention also relates to the use of the catalysts for catalysing the hydrogenation of carbon dioxide to methanol.

### BACKGROUND OF THE INVENTION

Due to increasing emissions by the ever-expanding global human population, the concentration of CO₂ in the atmosphere is rising year on year, and is widely accepted to be a key contributor in global climate change. Attempts to reduce CO₂ emissions are plentiful, as are CO₂ capture and transformation technologies. It has recently been demonstrated that by utilizing solar energy, wind power, hydropower and biomass, renewable hydrogen gas can be produced on a large scale^{1,2}. Therefore, the recycling of CO₂ through its hydrogenation to high-energy-content fuels such as alcohols or hydrocarbons appears to be very attractive³.

The hydrogenation of CO₂ to methanol has attracted significant interest due to the value of methanol, both as a chemical platform and a clean fuel. Compounds able to effectively catalyse the conversion of CO₂ to methanol are therefore becoming increasingly attractive.

Cu/ZnO-based catalysts are known to be active in the catalytic hydrogenation of CO₂ to methanol. Within such Cu/ZnO-based catalysts, the surface of Cu is generally accepted to provide catalytic active sites, although the role(s) of the ZnO support is still not clear⁴⁻¹¹. It has also been reported that the incorporation of different additives, such as Al₂O₃, ZrO₂, SiO₂ and Ga₂O₃ can further improve the activity, stability and thermal resistance compared with the unmodified Cu/ZnO catalyst¹²⁻¹⁷. Recently, using atom probe tomography technique, stable Cu-containing small and active crystallites (-0.5-2 nm) can be identified in the working catalyst prepared from Ga³⁺ modified Cu/ZnO¹⁸⁻²⁰. The formation of ZnGa₂O₄ spinel structure is believed to enhance the generation of extremely small Cu clusters under methanol synthesis conditions.

In spite of the advances made with Cu/ZnO-based catalysts, there remains a need for improved catalysts capable of catalyzing the hydrogenation of CO₂ to methanol.

The present invention was devised with the foregoing in mind.

### SUMMARY OF THE INVENTION

According to a first aspect of the present invention there is provided a particle having a silica core and a layered double hydroxide shell, wherein the layered double hydroxide comprises Cu²⁺ and Zn²⁺.

Suitably the particle is a plurality of particles (e.g. a powder), each having a silica core and a layered double hydroxide shell, wherein the layered double hydroxide comprises Cu²⁺ and Zn²⁺.

According to a second aspect of the present invention there is provided a process for preparing particles (e.g. particles of the first aspect), the process comprising the step of:
a) contacting:
   silica particles,
   an aqueous solution of layered double hydroxide-forming metal cations, said metal cations comprising Cu²⁺, Zn²⁺ and one or more trivalent or tetravalent metal cations, and
   an aqueous solution of one or more layered double hydroxide-forming anions, under basic conditions.

According to a third aspect of the present invention there is provided a particle obtained, directly obtained or obtainable by the process of the second aspect.

Suitably the particle is a plurality of particles (e.g. a powder).

According to a fourth aspect of the present invention there is provided a catalyst precursor, wherein the catalyst precursor is a thermally treated form of the particle of the first or third aspects.

According to a fifth aspect of the present invention there is provided a catalyst precursor in the form of particle having a silica core and a layered double oxide shell, wherein the layered double oxide comprises Cu²⁺ and Zn²⁺.

According to a sixth aspect of the present invention there is provided a process for the preparation of a catalyst precursor (e.g. a catalyst precursor of the fourth or fifth aspect), the process comprising the steps of:
a) providing a particle of the first or third aspects; and
b) thermally treating the particle provided in step a).

According to a seventh aspect of the present invention there is provided a catalyst precursor obtained, directly obtained or obtainable by the process of the sixth aspect.

According to a eighth aspect of the present invention there is provided a catalyst, wherein the catalyst is a reduced or partially reduced form of the catalyst precursor of the fourth, fifth or seventh aspects.

According to an nineth aspect of the present invention there is provided a catalyst in the form of a particle having a silica core and a shell formed from Al₂O₃ and ZnO, wherein the shell comprises either or both of free Cu and a free Cu-Zn alloy

According to an tenth aspect of the present invention there is provided a process for the preparation of a catalyst (e.g. a catalyst of the eighth or nineth aspect), the process comprising the steps of:
a) providing a catalyst precursor of the fourth, fifth or seventh aspect;
b) reducing (or partially reducing) the catalyst precursor provided in step a).

According to an eleventh aspect of the present invention there is provided a catalyst obtained, directly obtained or obtainable by the process of the tenth aspect.

According to an twelfth aspect of the present invention there is provided a process for the preparation of methanol, the process comprising the step of:
a) contacting a catalyst of the eighth, nineth or eleventh aspect with a mixture of hydrogen and carbon dioxide.

### DETAILED DESCRIPTION OF THE INVENTION

Throughout the entirety of the description and claims of this specification, where subject matter is described herein using the term "comprise" (or "comprises" or "comprising"), the same subject matter instead described using the term "consist of" (or "consists of" or "consisting of") or "consist essentially of" (or "consists essentially of" or "consisting essentially of") is also contemplated.

Throughout the description and claims of this specification, the singular encompasses the plural unless the context otherwise requires. In particular, where the indefinite article is used, the specification is to be understood as contemplating plurality as well as singularity, unless the context requires otherwise.

Features described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any of the specific embodiments recited herein. The invention extends to any novel one, or any novel combination, of the features disclosed in this specification (including any accompanying claims, abstract and drawings), or to any novel one, or any novel combination, of the steps of any method or process so disclosed.

Unless otherwise specified, where the quantity or concentration of a particular component of a given product is specified as a weight percentage (wt.% or %w/w), said weight percentage refers to the percentage of said component by weight relative to the total weight of the product as a whole. It will be understood by those skilled in the art that the sum of weight percentages of all components of a product will total 100 wt.%. However, where not all components are listed (e.g. where a product is said to "comprise" one or more particular components), the weight percentage balance may optionally be made up to 100 wt% by unspecified ingredients.

### Silica-LDH core-shell particles

As described hereinbefore, in a first aspect the present invention provides a particle having a silica core and a layered double hydroxide (LDH) shell, wherein the LDH comprises Cu²⁺ and Zn²⁺.

Through rigorous investigations, the inventors have surprisingly found that silica-LDH core-shell particles as described herein can serve as convenient starting materials for the preparation of new catalysts capable of hydrogenating CO₂ (or CO) to methanol. When compared with industry standard CO₂ to methanol catalysts, as well as those prepared from unsupported LDH, the catalysts obtainable from silica-LDH core-shell particles described herein exhibit notably improved catalytic properties, even at comparatively lower copper loading.

Although much of the discussion outlined herein refers to a particle of the first aspect, it will be understood that the particle of the first aspect may be provided as a plurality of particles, each having the core-shell arrangement described herein. Suitably, the particle is provided as a powder comprising a population of core-shell particles.

The structure of LDHs will be familiar to one of ordinary skill in the art as comprising a stack of positively charged, brucite-like layers of octahedral metal hydroxides intercalated by exchangeable, charge-balancing anions, where water typically provides hydrogen bonding between the positively charged layers. The layers of metal hydroxides are typically formed from a mixture of divalent and trivalent metal cations, although variants in which the divalent cation is replaced by (or supplemented with) a monovalent cation and/or the trivalent cation is replaced by (or supplemented with) a tetravalent cation are known. The LDHs used to prepare the particle of the invention comprise Cu²⁺ and Zn²⁺ (i.e. divalent copper and zinc cations) in the positively charged metal hydroxide layers.

The LDH may be such that the combined amounts of Cu²⁺ and Zn²⁺ accounts for at least 10 mol% of all monovalent and divalent metal cations present within the LDH. Suitably, the combined amounts of Cu²⁺ and Zn²⁺ accounts for at least 40 mol% of all monovalent and divalent metal cations present within the LDH. More suitably, the combined amounts of Cu²⁺ and Zn²⁺ accounts for at least 70 mol% of all monovalent and divalent metal cations present within the LDH. Even more suitably, the combined amounts of Cu²⁺ and Zn²⁺ accounts for at least 85 mol% of all monovalent and divalent metal cations present within the LDH. Most suitably, the combined amounts of Cu²⁺ and Zn²⁺ accounts for at least 90 mol% of all monovalent and divalent metal cations present within the LDH.

In particular embodiments, Cu²⁺ and Zn²⁺ may account for substantially all (or all) monovalent and divalent metal cations present within the LDH.

One or more of Mg²⁺, Ni²⁺ and Ca²⁺ may form the optional balance of all monovalent and divalent metal cations within the LDH. The term *optional balance* will be understood to refer to all non-Cu²⁺ and non-Zn²⁺ monovalent and divalent metal cations present within the LDH, recognising that in particular embodiments, Cu²⁺ and Zn²⁺ may account for substantially all (or all) monovalent and divalent metal cations present within the LDH. Accordingly, the term *optional* balance encompasses two alternatives: i) where Cu²⁺ and Zn²⁺ account for all monovalent and divalent metal cations present within the LDH (i.e., no balance of monovalent and divalent metal cations exists), and ii) where Cu²⁺ and Zn²⁺ do not account for all monovalent and divalent metal cations present within the LDH (i.e., a balance of monovalent and divalent metal cations exists). Suitably, Mg²⁺ may form the optional balance of all monovalent and divalent metal cations within the LDH.

As discussed hereinbefore, it will be understood that the LDH comprises at least one trivalent or tetravalent metal cation. One or more of Al³⁺, Ga³⁺, In³⁺ and Zr⁴⁺ may account for all trivalent and tetravalent metal cations present within the LDH. Suitably, one or more of Al³⁺, Ga³⁺ and In³⁺ accounts for all trivalent and tetravalent metal cations present within the LDH. More suitably, Al³⁺ accounts for at least 80 mol% of all trivalent and tetravalent metal cations present within the LDH. Most suitably, Al³⁺ accounts for all trivalent and tetravalent metal cations present within the LDH

In particular embodiments, Mg²⁺ forms the optional balance of all monovalent and divalent metal cations within the LDH and Al³⁺ accounts for at least 80 mol% (e.g. all) of the trivalent and tetravalent metal cations present within the LDH.

The molar ratio of Cu²⁺ to all trivalent and tetravalent metal cations (e.g. Al³⁺) present within the LDH may be 0.8 - 4.0. Suitably, the molar ratio of Cu²⁺ to all trivalent and tetravalent metal cations present within the LDH is 0.9 - 3.0. More suitably, the molar ratio of Cu²⁺ to all trivalent and tetravalent metal cations present within the LDH is 1.0 - 2.3. Even more suitably, the molar ratio of Cu²⁺ to all trivalent and tetravalent metal cations present within the LDH is 1.0 - 2.0. Yet even more suitably, the molar ratio of Cu²⁺ to all trivalent and tetravalent metal cations present within the LDH is 1.0 - 1.6. Most suitably, the molar ratio of Cu²⁺ to all trivalent and tetravalent metal cations present within the LDH is 1.2 - 1.4.

The molar ratio of Cu²⁺ to Zn²⁺ within the LDH may be 0.8 - 4.5. Suitably, the molar ratio of Cu²⁺ to Zn²⁺ within the LDH is 0.8 - 4.0. More suitably, the molar ratio of Cu²⁺ to Zn²⁺ within the LDH is 0.8 - 3.0. Even more suitably, the molar ratio of Cu²⁺ to Zn²⁺ within the LDH is 1.0 - 2.3. Yet more suitably, the molar ratio of Cu²⁺ to Zn²⁺ within the LDH is 1.0 - 2.0. Still even more suitably, the molar ratio of Cu²⁺ to Zn²⁺ within the LDH is 1.0 - 1.6. Most suitably, the molar ratio of Cu²⁺ to Zn²⁺ within the LDH is 1.2 - 1.4.

The molar ratio of Zn²⁺ to all trivalent and tetravalent metal cations (e.g. Al³⁺) present within the LDH may be 0.7 - 1.3. Suitably, the molar ratio of Zn²⁺ to all trivalent and tetravalent metal cations present within the LDH is 0.8 - 1.2. Most suitably, the molar ratio of Zn²⁺ to all trivalent and tetravalent metal cations present within the LDH is 0.9 - 1.1.

In particular embodiments, the molar ratio of Cu²⁺ to all trivalent and tetravalent metal cations (e.g. Al³⁺) present within the LDH is 1.0 - 2.3; the molar ratio of Cu²⁺ to Zn²⁺ within the LDH is 1.0 - 2.3; and the molar ratio of Zn²⁺ to all trivalent and tetravalent metal cations (e.g. Al³⁺) present within the LDH is 0.8 - 1.2. Suitably, the combined amounts of Cu²⁺ and Zn²⁺ accounts for at least 70 mol% (e.g. at least 90 mol%) of all monovalent and divalent metal cations present within the LDH.

As discussed hereinbefore, it will be understood that the LDH comprises at least one anion. One or more of an inorganic oxyanion, a halide and hydroxide may account for all anions present within the LDH interlayer. Exemplary halides include fluoride, chloride and bromide, of which chloride is preferred. Exemplary inorganic oxyanions include carbonate, bicarbonate, nitrate, nitrite, sulfate, borate and phosphate, of which carbonate is preferred. Suitably, an inorganic oxyanion accounts for all anions present within the LDH interlayer. Most suitably, carbonate accounts for all anions present within the LDH interlayer.

In particular embodiments, Mg²⁺ forms the optional balance of all monovalent and divalent metal cations within the LDH; Al³⁺ accounts for all trivalent and tetravalent metal cations present within the LDH; and an inorganic oxyanion (e.g. carbonate) accounts for all anions present within the LDH interlayer.

The LDH may have a composition according to formula (I):

[(Cu²⁺ₓZn²⁺_{y}M²⁺_{z})_{1-*q*}M³⁺*_{q}*(OH)₂]^{*q*+}(X^{*n*-})_{*q*/*n*}·*w*(H₂O)·*u*(OS) (I)

wherein
M²⁺ is at least one divalent metal cation;
M³⁺ is at least one trivalent metal cation;
0.1≤ (*x*+*y*) ≤1;
*z* = 1 - (*x*+*y*);
0<*q*<0.9;
0≤*w*≤10;
0≤*u*≤10
X is an anion;
OS is an organic solvent capable of hydrogen bonding to water; and
*n* is the charge on anion X.

M²⁺ may be one or more of Mg²⁺, Ni²⁺ and Ca²⁺. Suitably, M²⁺ is Mg²⁺.

M³⁺ may be one or more of Al³⁺, Ga³⁺ and In³⁺. Suitably, M³⁺ is Al³⁺.

X may be one or more selected from a halide, an inorganic oxyanion and hydroxide. Suitably, X is an inorganic oxyanion. Most suitably, X is carbonate.

In particular embodiments, M²⁺ is Mg²⁺, M³⁺ is Al³⁺ and X is an inorganic oxyanion (e.g. carbonate.)

It may be such that 0.4≤(*x*+*y*)≤1. Suitably, 0.7≤(*x*+*y*)≤1. Most suitably, 0.9≤(*x*+*y*)≤1. In particular embodiments, *x*+*y* = 1 (or substantially 1).

It may be such that 0.1≤*q*≤0.5. Suitably, 0.2≤*q*≤0.4.

It may be such that *x*(*1-q*)/*q* = 0.8 - 4.0. Suitably, *x*(1-*q*)/*q* = 0.9 - 3.0. More suitably, *x*(1-*q*)/*q* = 1.0 - 2.3. Even more suitably, *x*(1-*q*)/*q* = 1.0 - 2.0. Yet even more suitably, *x*(1-*q*)/*q* = 1.0 - 1.6. Most suitably, *x*(1-*q*)/*q* = 1.2 - 1.4

It may be such that *x*/*y* = 0.8 - 4.5. Suitably, *x*/*y* = 0.8 - 4.0. More suitably, *x*/*y* = 0.8 - 3.0. Even more suitably, *x*/*y* = 1.0 - 2.3. Yet even more suitably, *x*/*y* = 1.0 - 2.0. Still even more suitably, *x*/*y* = 1.0 - 1.6. Most suitably, *xly* = 1.2 - 1.4.

It may be such that *y*(*1-q*)/*q* = 0.7 - 1.3. Suitably, *y*(*1-q*)/*q* = 0.8 - 1.2. Most suitably, *y*(1-*q*)/*q* = 0.9 - 1.1.

In particular embodiments, *x*(1-*q*)/*q* = 1.0 - 2.3; *x*/*y* = 1.0 - 2.3; and *y*(*1-q*)/*q* = 0.8 - 1.2. Suitably, 0.7≤(*x*+*y*)≤1 (e.g. 0.9≤(*x*+*y*)≤1).

OS may be an organic solvent capable of donating hydrogen bonds to, or accepting hydrogen bonds from, water. Organic solvents containing hydrogen bond donating and accepting moieties will be readily familiar to one of ordinary skill in the art. Particular, non-limiting classes of organic solvents capable of hydrogen bonding to water are alcohols, ketones and aldehydes. Suitably, OS is one or more selected from acetone, ethanol, methanol and 1-hexanol. In particular embodiments, OS is ethanol.

LDHs in which *u*>0 have physical properties that may make them particularly suitable for use in the preparation of catalysts. In particular, such LDHs may exhibit an increased surface area, increased pore volume and/or a reduced density relative to LDHs in which *u* = 0.

LDHs in which *u*>0 can be readily prepared by one of skill in the art. For example, such LDHs can be produced by first precipitating a LDH from an aqueous (i.e. water-containing) solvent, and then contacting (e.g. by washing or dispersing) the resulting, water-wet LDH with one or more organic solvents, OS as defined herein. The term *water-wet* will be understood to mean that the precipitated LDH is not allowed to become dry before it is contacted with the one or more organic solvents, OS. The precipitated LDH may be washed with water before it is contacted with (e.g. washed with or dispersed in) the one or more organic solvents, OS.

In particular embodiments, *u* = 0.

The silica core may account for 10 - 50 wt% of the particle and the LDH may account for 50 - 90 wt% of the particle. Suitably, the silica core accounts for 20 - 50 wt% of the particle and the LDH accounts for 50 - 80 wt% of the particle. More suitably, the silica core accounts for 30 - 50 wt% of the particle and the LDH accounts for 50 - 70 wt% of the particle. Most suitably, the silica core accounts for 35 - 45 wt% of the particle and the LDH accounts for 55 - 65 wt% of the particle.

In particular embodiments, the combined amounts of Cu²⁺ and Zn²⁺ accounts for at least 70 mol% (e.g. at least 90 mol%) of all monovalent and divalent metal cations present within the LDH; and the silica core accounts for 30 - 50 wt% of the particle and the LDH accounts for 50 - 70 wt% of the particle. Suitably, the molar ratio of Cu²⁺ to all trivalent and tetravalent metal cations (e.g. Al³⁺) present within the LDH is 1.0 - 2.3 and/or the molar ratio of Cu²⁺ to Zn²⁺ within the LDH is 1.0 - 2.3 and/or the molar ratio of Zn²⁺ to all trivalent and tetravalent metal cations (e.g. Al³⁺) present within the LDH is 0.8 - 1.2.

In particular embodiments, the silica core accounts for 30 - 50 wt% of the particle and the LDH accounts for 50 - 70 wt% of the particle; Mg²⁺ forms the optional balance of all monovalent and divalent metal cations within the LDH; Al³⁺ accounts for all trivalent and tetravalent metal cations present within the LDH; and an inorganic oxyanion (e.g. carbonate) accounts for all anions present within the LDH interlayer. Suitably, the combined amounts of Cu²⁺ and Zn²⁺ accounts for at least 70 mol% (e.g. at least 90 mol%) of all monovalent and divalent metal cations present within the LDH.

The LDH shell may have an average thickness of 0.001 - 1.5 µm, as determined by SEM or TEM. Suitably, the LDH shell has an average thickness of 0.03 - 0.5 µm (e.g. 0.03 - 0.45 µm).

The silica core may have an average particle size of 0.1 - 500 µm, as determined by SEM or TEM. It will be understood that references made herein to the average size of particles refer to the average diameter of those particles. Suitably, the silica core has an average particle size of 0.2 - 100 µm (e.g. 0.2 - 75 µm). More suitably, the silica core has an average particle size of 0.3 - 60 µm (e.g. 0.3 - 50 µm). In any of the aforementioned particle size ranges, the lower limit may alternatively be 1 µm.

The particle may further comprise an interface layer disposed between the silica core and the LDH shell. The interface layer may comprise a metal silicate and/or a metal hydroxyoxyanion (e.g. a metal hydroxycarbonate, a metal hydroxynitrate or a metal hydroxysulphate).

As described hereinbefore, in a second aspect the present invention provides a process for preparing particles (e.g. particles of the first aspect), the process comprising the step of:
a) contacting:
   silica particles,
   an aqueous solution of LDH-forming metal cations, said metal cations comprising Cu²⁺, Zn²⁺ and one or more trivalent or tetravalent metal cations, and
   an aqueous solution of one or more LDH-forming anions,
      under basic conditions.

The inventors have devised a process for preparing silica-LDH core-shell particles that can serve as convenient starting materials for the preparation of new catalysts capable of hydrogenating CO₂ (or CO) to methanol. The process involves coprecipitating a Cu²⁺- and Zn²⁺-containing LDH in the presence of silica particles, such that the LDH grows as a shell around the silica particles. The skilled person will be familiar with coprecipitation techniques for forming LDHs.

The process may further comprise a step (step b)) of aging the mixture resulting from step
a). The aging step may proceed for a period of 0.1 - 48 hours.

The process may further comprise a step (step c)) of isolating the particles resulting from step a) or b) (if applicable). The particles may, for example, be isolated by filtration, followed by washing (e.g. with water) and then drying (e.g. vacuum drying). The isolation process may comprise, prior to drying, a step of contacting (e.g. rinsing, washing or immersing) the still water-wet (e.g. damp) LDH with an organic solvent capable of hydrogen bonding to water (e.g. one of those defined hereinbefore).

The basic conditions may be such that the mixture formed in step a) has a pH of 8.5 - 11.5. Suitably, the basic conditions are such that the mixture formed in step a) has a pH of 8.5 - 10.5. Most suitably, the basic conditions are such that the mixture formed in step a) has a pH of 8.7 - 9.3. The inventors have determined that using a pH in this range gives rise to excellent (e.g. uniform) shell morphology.

It will be understood that the particles prepared according to the second aspect can have any of those properties or characteristics recited hereinbefore in relation to the first aspect.

As described hereinbefore, in a third aspect the present invention provides a particle obtained, directly obtained or obtainable by the process of the second aspect.

### Catalyst precursors

As described hereinbefore, in a fourth aspect the present invention provides a catalyst precursor, wherein the catalyst precursor is a thermally treated form of the particle of the first or third aspects.

The particles of the first and third aspects can be straightforwardly converted into a precursor for a CO₂ to methanol catalyst by a simple thermal treatment technique.

The thermally treated form of the particle may be a calcined form of the particle. The skilled person will be familiar with the calcination of LDHs to afford layered double oxides (LDOs).

As described hereinbefore, in a fifth aspect the present invention provides a catalyst precursor in the form of a particle having a silica core and a LDO shell, wherein the LDO comprises Cu²⁺ and Zn²⁺.

The LDO may be such that the combined amounts of Cu²⁺ and Zn²⁺ accounts for at least 10 mol% of all monovalent and divalent metal cations present within the LDO. Suitably, the combined amounts of Cu²⁺ and Zn²⁺ accounts for at least 40 mol% of all monovalent and divalent metal cations present within the LDO. More suitably, the combined amounts of Cu²⁺ and Zn²⁺ accounts for at least 70 mol% of all monovalent and divalent metal cations present within the LDO. Most suitably. the combined amounts of Cu²⁺ and Zn²⁺ accounts for at least 90 mol% of all monovalent and divalent metal cations present within the LDO.

In particular embodiments, Cu²⁺ and Zn²⁺ may account for substantially all (or all) monovalent and divalent metal cations present within the LDO.

One or more of Mg²⁺, Ni²⁺ and Ca²⁺ may form the optional balance of all monovalent and divalent metal cations within the LDO. The term *optional balance* will be understood to refer to all non-Cu²⁺ and non-Zn²⁺ monovalent and divalent metal cations present within the LDO, recognising that in particular embodiments, Cu²⁺ and Zn²⁺ may account for substantially all (or all) monovalent and divalent metal cations present within the LDO. Suitably, Mg²⁺ may form the optional balance of all monovalent and divalent metal cations within the LDO.

As discussed hereinbefore, it will be understood that the LDO comprises at least one trivalent or tetravalent metal cation. One or more of Al³⁺, Ga³⁺, In³⁺ and Zr⁴⁺ may account for all trivalent and tetravalent metal cations present within the LDO. Suitably, one or more of Al³⁺, Ga³⁺ and In³⁺ accounts for all trivalent and tetravalent metal cations present within the LDO. More suitably, Al³⁺ accounts for at least 80 mol% of all trivalent and tetravalent metal cations present within the LDO. Most suitably, Al³⁺ accounts for all trivalent and tetravalent metal cations present within the LDO.

In particular embodiments, Mg²⁺ forms the optional balance of all monovalent and divalent metal cations within the LDO and Al³⁺ accounts for at least 80 mol% (e.g. all) of the trivalent and tetravalent metal cations present within the LDO.

The molar ratios of metal cations within the LDO may be identical or substantially identical to those of the LDH forming part of the invention. Accordingly the molar ratio of Cu²⁺ to all trivalent and tetravalent metal cations present within the LDO and/or the molar ratio of Cu²⁺ to Zn²⁺ within the LDO and/or the molar ratio of Zn²⁺ to all trivalent and tetravalent metal cations present within the LDO may have any of those definitions recited hereinbefore in relation to the first aspect.

The catalyst precursor particle may further comprise an interface layer disposed between the silica core and the LDO shell. The interface layer may comprise one or more metal oxides.

The silica core may account for 15 - 60 wt% of the catalyst precursor particle and the LDO may account for 40 - 85 wt% of the catalyst precursor particle. Suitably, the silica core accounts for 27 - 60 wt% of the particle and the LDO accounts for 40 - 73 wt% of the particle. More suitably, the silica core accounts for 40 - 60 wt% of the particle and the LDO accounts for 40 - 60 wt% of the particle. Most suitably, the silica core accounts for 45 - 55 wt% of the particle and the LDO accounts for 45 - 55 wt% of the particle.

In particular embodiments, the combined amounts of Cu²⁺ and Zn²⁺ accounts for at least 70 mol% (e.g. at least 90 mol%) of all monovalent and divalent metal cations present within the LDO; and the silica core accounts for 30 - 50 wt% of the particle and the LDO accounts for 50 - 70 wt% of the particle. Suitably, the molar ratio of Cu²⁺ to all trivalent and tetravalent metal cations (e.g. Al³⁺) present within the LDO is 1.0 - 2.3 and/or the molar ratio of Cu²⁺ to Zn²⁺ within the LDO is 1.0 - 2.3 and/or the molar ratio of Zn²⁺ to all trivalent and tetravalent metal cations (e.g. Al³⁺) present within the LDO is 0.8 - 1.2.

The LDO shell may have an average thickness of 0.001 - 1.5 µm, as determined by SEM or TEM. Suitably, the LDO shell has an average thickness of 0.03 - 0.5 µm (e.g. 0.03 - 0.45 µm).

The silica core may have an average particle size of 0.1 - 500 µm, as determined by SEM or TEM. Suitably, the silica core has an average particle size of 0.2 - 100 µm (e.g. 0.2 - 75 µm). More suitably, the silica core has an average particle size of 0.3 - 60 µm (e.g. 0.3 - 50 µm). In any of the aforementioned particle size ranges, the lower limit may alternatively be 1 µm.

As described hereinbefore, in a sixth aspect the present invention provides a process for the preparation of a catalyst precursor (e.g. a catalyst precursor of the fourth or fifth aspect), the process comprising the steps of:
a) providing a particle of the first or third aspects; and
b) thermally treating the particle provided in step a).

Step b) may comprise thermally treating the particle provided in step a) at a temperature of 200 - 550°C. Suitably, step b) comprises thermally treating the particle provided in step a) at a temperature of 280 - 380°C. The skilled person will be familiar with calcination techniques, such as step b).

As described hereinbefore, in a seventh aspect the present invention provides a catalyst precursor obtained, directly obtained or obtainable by the process of the sixth aspect.

It will be understood that the particles prepared according to the sixth or seventh aspect can have any of those properties or characteristics recited hereinbefore in relation to the fourth or fifth aspect.

### Catalysts

As described hereinbefore, in a nineth aspect the present invention provides a catalyst in the form of a particle having a silica core and a shell comprising Al₂O₃ and ZnO, wherein the shell comprises either or both of free Cu and a free Cu-Zn alloy.

When compared with industry standard CO₂ to methanol catalysts, as well as those prepared from unsupported LDH, the catalysts of the invention exhibit notably improved catalytic properties, even at comparatively lower copper loading.

The term *free* used herein in relation to Cu and Cu-Zn alloys refers to those species being present in a metallic state (e.g. having an oxidation state of zero).

The free Cu and/or free Cu-Zn alloy may be in the form of particles. Suitably, the particles have an average size of 1 - 30 nm as determined by SEM or TEM. More suitably, the particles have an average size of 3 - 15 nm (e.g. 3-7 nm).

The particles may have a crystallite domain size of 1 - 15 nm as calculated by Rietveld refinement of XRD data. Suitably, the particles have a crystallite domain size of 1 - 7 nm.

The catalyst may comprise 10 - 45 wt.% of Cu.

The catalyst may comprise 5 - 25 wt.% of Zn.

The catalyst may comprise 1 - 12 wt.% of Al.

In particular embodiments, the catalyst comprises 10 - 45 wt.% of Cu, 5 - 25 wt.% of Zn and 1 - 12 wt.% of Al.

The molar ratios of metals and cations thereof within the catalyst may be identical or substantially identical to those of the LDH and/or LDO forming part of the invention.

The molar ratio of Cu to Al within the catalyst may be 0.8 - 4.0. Suitably, the molar ratio of Cu to Al within the catalyst is 0.9 - 3.0. More suitably, the molar ratio of Cu to Al within the catalyst is 1.0 - 2.3. Even more suitably, the molar ratio of Cu to Al within the catalyst is 1.0 - 2.0. Yet even more suitably, the molar ratio of Cu to Al within the catalyst is 1.0 - 1.6. Most suitably, the molar ratio of Cu to Al within the catalyst is 1.2 - 1.4.

The molar ratio of Cu to Zn within the catalyst may be 0.8 - 4.5. Suitably, the molar ratio of Cu to Zn within the catalyst is 0.8 - 4.0. More suitably, the molar ratio of Cu to Zn within the catalyst is 0.8 - 3.0. Even more suitably, the molar ratio of Cu to Zn within the catalyst is 1.0 - 2.3. Yet more suitably, the molar ratio of Cu to Zn within the catalyst is 1.0 - 2.0. Still even more suitably, the molar ratio of Cu to Zn within the catalyst is 1.0 - 1.6. Most suitably, the molar ratio of Cu to Zn within the catalyst is 1.2 - 1.4.

The molar ratio of Zn to Al within the catalyst may be 0.7 - 1.3. Suitably, the molar ratio of Zn to Al within the catalyst is 0.8 - 1.2. Most suitably, the molar ratio of Zn to Al within the catalyst is 0.9 - 1.1.

In particular embodiments, the molar ratio of Cu to Al within the catalyst is 1.0 - 2.3; the molar ratio of Cu to Zn within the catalyst is 1.0 - 2.3; and the molar ratio of Zn to Al within the catalyst is 0.8 - 1.2. Suitably, the catalyst comprises 10 - 45 wt.% of Cu and/or 5-25 wt.% of Zn and/or 1 - 12 wt.% of Al.

The catalyst may have a Cu dispersion of 5 - 30%, as determined by N₂O chemisorption followed by hydrogen reduction. Suitably, the catalyst has a Cu dispersion of 10 - 27%.

The catalyst may have a specific surface area of Cu (S_{Cu}) of 15 - 35 m²/g_{cat}, as determined by N₂O chemisorption results.

The silica core may account for 20 - 62 wt% of the catalyst particle and the shell may account for 38 - 80 wt% of the catalyst particle. Suitably, the silica core accounts for 32 - 62 wt% of the particle and the shell accounts for 38 - 68 wt% of the particle. More suitably, the silica core accounts for 43 - 62 wt% of the particle and the shell accounts for 38 - 57 wt% of the particle. Most suitably, the silica core accounts for 45 - 60 wt% of the particle and the shell accounts for 40 - 55 wt% of the particle.

The shell may have an average thickness of 0.001 - 1.5 µm, as determined by SEM or TEM. Suitably, the shell has an average thickness of 0.03 - 0.5 µm (e.g. 0.03 - 0.45 µm).

The silica core may have an average particle size of 0.1 - 500 µm, as determined by SEM or TEM. Suitably, the silica core has an average particle size of 0.2 - 100 µm (e.g. 0.2 - 75 µm). More suitably, the silica core has an average particle size of 0.3 - 60 µm (e.g. 0.3 - 50 µm). In any of the aforementioned particle size ranges, the lower limit may alternatively be 1 µm.

As described hereinbefore, in an eighth aspect the present invention provides a catalyst, wherein the catalyst is a reduced or partially reduced form of the catalyst precursor of the fourth, fifth or seventh aspects.

As described hereinbefore, in a tenth aspect the present invention provides a process for the preparation of a catalyst (e.g. a catalyst of the eighth or nineth aspect), the process comprising the steps of:
a) providing a catalyst precursor of the fourth, fifth or seventh aspect;
b) reducing the catalyst precursor provided in step a).

The skilled person will be familiar with processes for reducing (or partially reducing) catalyst precursors to produce a working catalyst.

Step b) may comprise heating the catalyst precursor in an atmosphere of hydrogen, optionally in the presence of nitrogen and/or argon (or another inert gas). Most suitably, step b) comprises heating the catalyst precursor to a temperature of 250-350°C in an atmosphere of hydrogen.

As described hereinbefore, in an eleventh aspect the present invention provides a catalyst obtained, directly obtained or obtainable by the process of the tenth aspect.

It will be understood that the catalyst prepared according to the eighth, tenth or eleventh aspect can have any of those properties or characteristics recited hereinbefore in relation to the nineth aspect.

### Catalytic processes

As described hereinbefore, in a twelfth aspect the present invention provides a process for the preparation of methanol, the process comprising the step of:
a) contacting a catalyst of the eighth, nineth or eleventh aspect with a mixture of hydrogen and carbon dioxide.

The mixture of hydrogen and carbon dioxide may comprise an excess of hydrogen relative to carbon dioxide. Suitably, the molar ratio of the mixture of hydrogen to carbon dioxide is (1.5 - 5):1. More suitably, the molar ratio of the mixture of hydrogen to carbon dioxide is (2 - 4):1.

Step a) may be conducted at a temperature of 200 - 380°C. Most suitably, step a) is conducted at a temperature of 240-320°C.

Step a) may be conducted at increased pressure, such as 20 - 60 bar. Most suitably, step a) is conducted as 35 - 55 bar (e.g. 40 - 50 bar).

In particular embodiments, step a) is conducted at a temperature of 240-320°C and the molar ratio of the mixture of hydrogen to carbon dioxide is (1.5 - 5):1 (e.g. (2 - 4):1).

The following numbered statements 1 to 91 are not claims, but instead describe particular aspects and embodiments of the invention:
1. A particle having a silica core and a layered double hydroxide shell, wherein the layered double hydroxide comprises Cu²⁺ and Zn²⁺.
2. The particle of statement 1, wherein the combined amounts of Cu²⁺ and Zn²⁺ accounts for at least 10 mol% or at least 40 mol% of all monovalent and divalent metal cations present within the layered double hydroxide.
3. The particle of statement 1, wherein the combined amounts of Cu²⁺ and Zn²⁺ accounts for at least 70 mol% or at least 90 mol% of all monovalent and divalent metal cations present within the layered double hydroxide.
4. The particle of statement 1, 2 or 3, wherein one or more of Mg²⁺, Ni²⁺ and Ca²⁺ form the optional balance of all monovalent and divalent metal cations within the layered double hydroxide.
5. The particle of any one of the preceding statements, wherein Mg²⁺ forms the optional balance of all monovalent and divalent metal cations within the layered double hydroxide.
6. The particle of any one of the preceding statements, wherein one or more of Al³⁺, Ga³⁺, In³⁺ and Zr⁴⁺ accounts for all trivalent and tetravalent metal cations present within the layered double hydroxide.
7. The particle of any one of the preceding statements, wherein Al³⁺ accounts for all trivalent and tetravalent metal cations present within the layered double hydroxide.
8. The particle of any one of the preceding statements, wherein the molar ratio of Cu²⁺ to all trivalent and tetravalent metal cations present within the layered double hydroxide is 0.8 - 4.0.
9. The particle of any one of the preceding statements, wherein the molar ratio of Cu²⁺ to all trivalent and tetravalent metal cations present within the layered double hydroxide is 1.0 - 2.3.
10. The particle of any one of the preceding statements, wherein the molar ratio of Cu²⁺ to all trivalent and tetravalent metal cations present within the layered double hydroxide is 1.0 - 2.0.
11. The particle of any one of the preceding statements, wherein the molar ratio of Cu²⁺ to all trivalent and tetravalent metal cations present within the layered double hydroxide is 1.0 - 1.6.
12. The particle of any one of the preceding statements, wherein the molar ratio of Cu²⁺ to Zn²⁺ within the layered double hydroxide is 0.8 - 4.5.
13. The particle of any one of the preceding statements, wherein the molar ratio of Cu²⁺ to Zn²⁺ within the layered double hydroxide is 0.8 - 2.0.
14. The particle of any one of the preceding statements, wherein the molar ratio of Cu²⁺ to Zn²⁺ within the layered double hydroxide is 1.0 - 1.6.
15. The particle of any one of the preceding statements, wherein the molar ratio of Zn²⁺ to all trivalent and tetravalent metal cations present within the layered double hydroxide is 0.7 - 1.3.
16. The particle of any one of the preceding statements, wherein the molar ratio of Zn²⁺ to all trivalent and tetravalent metal cations present within the layered double hydroxide is 0.8 - 1.2.
17. The particle of any one of the preceding statements, wherein one or more of an inorganic oxyanion, a halide and hydroxide accounts for all anions present within the layered double hydroxide interlayer.
18. The particle of any one of the preceding statements, wherein an inorganic oxyanion accounts for all anions present within the layered double hydroxide interlayer.
19. The particle of any one of the preceding statements, wherein the inorganic oxyanion is carbonate.
20. The particle of statement 1, wherein the layered double hydroxide comprising Cu²⁺ and Zn²⁺ is of formula (I):

   [(Cu²⁺ₓZn²⁺_{y}M²⁺_{z})_{1*-q*}M³⁺*_{q}*(OH)₂]^{*q*+}(X^{*n*-})_{*q*/n}·*w*(H₂O)·*u*(OS) (I)

   wherein
   M²⁺ is at least one divalent metal cation;
   M³⁺ is at least one trivalent metal cation;
   0.1≤ (*x*+*y*) ≤1;
   *z* = 1 - (*x*+*y*);
   0<*q*<0.9;
   0≤*w*≤10;
   0≤*u*≤10
   X is an anion;
   OS is an organic solvent capable of hydrogen bonding to water; and
   *n* is the charge on anion X.
21. The particle of statement 20, wherein M²⁺ is one or more of Mg²⁺, Ni²⁺ and Ca²⁺.
22. The particle of statement 20, wherein M²⁺ is Mg²⁺.
23. The particle of statement 20, 21 or 22, wherein M³⁺ is one or more of Al³⁺, Ga³⁺ and In³⁺.
24. The particle of statement 23, wherein M³⁺ is Al³⁺.
25. The particle of any one of statements 20 to 24, wherein X is one or more selected from a halide, an inorganic oxyanion and hydroxide.
26. The particle of any one of statements 20 to 24, wherein X is an inorganic oxyanion.
27. The particle of statement 25 or 26, wherein the inorganic oxyanion is carbonate.
28. The particle of statement 20, wherein M²⁺ is Mg²⁺, M³⁺ is Al³⁺ and X is carbonate.
29. The particle of any one of statements 20 to 28, wherein 0.4≤ (*x*+*y*) ≤1.
30. The particle of any one of statements 20 to 29, wherein 0.9≤ (*x*+*y*) ≤1.
31. The particle of any one of statements 20 to 30, wherein 0.1≤*q*≤0.5.
32. The particle of any one of statements 20 to 31, wherein 0.2≤*q*≤0.4.
33. The particle of any one of statements 20 to 32, wherein *x*(*1-q*)/*q* = 0.8 - 4.0.
34. The particle of any one of statements 20 to 33, wherein *x*/*y* = 0.8 - 4.5.
35. The particle of any one of statements 20 to 34, wherein *y*(*1-q*)/*q* = 0.7 - 1.3.
36. The particle of any one of the preceding statements, wherein the silica core accounts for 10 - 50 wt% of the particle and the layered double hydroxide accounts for 50 - 90 wt% of the particle.
37. The particle of any one of the preceding statements, wherein the silica core accounts for 20 - 50 wt% of the particle and the layered double hydroxide accounts for 50 - 80 wt% of the particle.
38. The particle of any one of the preceding statements, wherein the silica core accounts for 30 - 50 wt% of the particle and the layered double hydroxide accounts for 50 - 70 wt% of the particle.
39. The particle of any one of the preceding statements, wherein the silica core accounts for 35 - 45 wt% of the particle and the layered double hydroxide accounts for 55 - 65 wt% of the particle.
40. The particle of any one of the preceding statements, wherein the layered double hydroxide shell has an average thickness of 0.001 - 1.5 µm.
41. The particle of any one of the preceding statements, wherein the layered double hydroxide shell has an average thickness of 0.03 - 0.5 µm.
42. The particle of any one of statements 1 to 35, wherein the silica core accounts for 30 - 50 wt% of the particle and the layered double hydroxide accounts for 50 - 70 wt% of the particle; and the layered double hydroxide shell has an average thickness of 0.03 - 0.45 µm.
43. The particle of any one of the preceding statements, wherein the silica core has an average particle size of 0.1 - 500 µm.
44. The particle of any one of the preceding statements, wherein the silica core has an average particle size of 0.2 - 100 µm.
45. The particle of any one of the preceding statements, wherein the silica core has an average particle size of 0.3 - 60 µm.
46. The particle of any one of the preceding statements, further comprising an interface layer disposed between the silica core and the layered double hydroxide shell.
47. The particle of statement 46, wherein the interface layer comprises a metal silicate and/or a metal hydroxyoxyanion (e.g. a metal hydroxycarbonate, a metal hydroxynitrate or a metal hydroxysulphate).
48. A process for preparing particles as claimed in any one of the preceding statements, the process comprising the step of:
   a) contacting:
      silica particles,
      an aqueous solution of layered double hydroxide-forming metal cations, said metal cations comprising Cu²⁺, Zn²⁺ and one or more trivalent or tetravalent metal cations, and
      an aqueous solution of one or more layered double hydroxide-forming anions,
         under basic conditions.
49. The process of statement 48, further comprising the step of:
   b) aging the mixture resulting from step a) for a period of 0.1 - 48 hours.
50. The process of statement 48 or 49, further comprising the step of: c) isolating the particles resulting from step a) or b).
51. The process of statement 48, 49 or 50, wherein the basic conditions are such that the mixture formed in step a) has a pH of 8.5 - 11.5.
52. The process of statement 48, 49 or 50, wherein the basic conditions are such that the mixture formed in step a) has a pH of 8.5 - 10.5.
53. The process of statement 48, 49 or 50, wherein the basic conditions are such that the mixture formed in step a) has a pH of 8.7 - 9.3.
54. A catalyst precursor, wherein the catalyst precursor is a thermally treated form of the particle as defined in any one of statements 1 to 47.
55. The catalyst precursor of statement 54, wherein the catalyst precursor is a calcined form of the particle as defined in any one of statements 1 to 47.
56. A catalyst precursor in the form of particle having a silica core and a layered double oxide shell, wherein the layered double oxide comprises Cu²⁺ and Zn²⁺.
57. The catalyst precursor of statement 56, wherein the combined amounts of Cu²⁺ and Zn²⁺ accounts for at least 40 mol% of all monovalent and divalent metal cations present within the layered double oxide.
58. The catalyst precursor of statement 56, wherein the combined amounts of Cu²⁺ and Zn²⁺ accounts for at least 90 mol% of all monovalent and divalent metal cations present within the layered double oxide.
59. The catalyst precursor of statement 50, 57 or 58, wherein one or more of Mg²⁺, Ni²⁺ and Ca²⁺ form the optional balance of all monovalent and divalent metal cations within the layered double oxide.
60. The catalyst precursor of any one statements 56 to 59, wherein Mg²⁺ forms the optional balance of all monovalent and divalent metal cations within the layered double oxide.
61. The catalyst precursor of any one of statements 56 to 60, wherein one or more of Al³⁺, Ga³⁺ and In³⁺ accounts for all trivalent and tetravalent metal cations present within the layered double oxide.
62. The catalyst precursor of any one of statements 56 to 61, wherein Al³⁺ accounts for all trivalent and tetravalent metal cations present within the layered double oxide.
63. The catalyst precursor of any one of statements 56 to 62, wherein the molar ratio of Cu²⁺ to all trivalent and tetravalent metal cations present within the layered double oxide is 0.8 -4.0.
64. The catalyst precursor of any one of statements 56 to 63, wherein the molar ratio of Cu²⁺ to all trivalent and tetravalent metal cations present within the layered double oxide is 1.0 -2.3.
65. The catalyst precursor of any one of statements 56 to 64, wherein the molar ratio of Cu²⁺ to all trivalent and tetravalent metal cations present within the layered double oxide is 1.0 -2.0.
66. The catalyst precursor of any one of statements 56 to 65, wherein the molar ratio of Cu²⁺ to all trivalent and tetravalent metal cations present within the layered double oxide is 1.0 -1.6.
67. The catalyst precursor of any one of statements 56 to 66, wherein the molar ratio of Cu²⁺ to Zn²⁺ within the layered double oxide is 0.8 - 4.5.
68. The catalyst precursor of any one of statements 56 to 67, wherein the molar ratio of Cu²⁺ to Zn²⁺ within the layered double oxide is 0.8 - 2.0.
69. The catalyst precursor of any one of statements 56 to 68, wherein the molar ratio of Cu²⁺ to Zn²⁺ within the layered double oxide is 1.0 - 1.6.
70. The catalyst precursor of any one of statements 56 to 69, wherein the molar ratio of Zn²⁺ to all trivalent and tetravalent metal cations present within the layered double oxide is 0.7 - 1.3.
71. The catalyst precursor of any one of statements 56 to 70, wherein the molar ratio of Zn²⁺ to all trivalent and tetravalent metal cations present within the layered double oxide is 0.8 - 1.2.
72. A process for the preparation of a catalyst precursor, the process comprising the steps of:
   a) providing a particle as defined in any one of statements 1 to 47; and
   b) thermally treating the particle provided in step a).
73. The process of statement 72, wherein step b) is conducted at a temperature of 200-550°C.
74. A catalyst, wherein the catalyst is a reduced or partially reduced form of the catalyst precursor of any one of statements 54 to 71.
75. A catalyst in the form of a particle having a silica core and a shell formed from Al₂O₃ and ZnO, wherein the shell comprises either or both of free Cu and a free Cu-Zn alloy.
76. The catalyst of statement 74 or 75, wherein the molar ratio of Cu to Zn is 0.8 - 4.5.
77. The catalyst of statement 74, 75 or 76, wherein the molar ratio of Cu to Al is 0.8 - 4.0.
78. The catalyst of any one of statements 74 to 77, wherein the free Cu and the free Cu-Zn alloy are in the form of particles having an average size of 1 - 30 nm.
79. The catalyst of any one of statements 74 to 77, wherein the free Cu and the free Cu-Zn alloy are in the form of particles having an average size of 3 - 15 nm.
80. The catalyst of any one of statements 74 to 77, wherein the free Cu and the free Cu-Zn alloy are in the form of particles having a crystallite domain size of 1 - 7 nm.
81. The catalyst of any one of statements 74 to 80, wherein the catalyst comprises 10-45 wt.% of Cu.
82. The catalyst of any one of statements 74 to 81, wherein the catalyst comprises 5 - 25 wt.% of Zn.
83. The catalyst of any one of statements 74 to 82, wherein the catalyst comprises 1 - 12 wt.% of Al.
84. The catalyst of any one of statements 74 to 83, wherein the Cu dispersion of the catalyst is 5 - 30%.
85. The catalyst of any one of statements 74 to 84, wherein the specific surface area of Cu (S_{Cu}) is 15 - 35 m²/g_{cat}.
86. A process for the preparation of a catalyst, the process comprising the steps of:
   a) providing a catalyst precursor of any one of statements 54 to 71;
   b) reducing the catalyst precursor provided in step a).
87. The process of statement 86, wherein step b) comprises heating the catalyst precursor in an atmosphere of hydrogen, optionally in the presence of nitrogen and/or argon.
88. The process of statement 86 or 87, wherein step b) comprises heating the catalyst precursor to a temperature of 250-350°C in an atmosphere of hydrogen.
89. A process for the preparation of methanol, the process comprising the step of:
   a) contacting a catalyst as defined in any one of statements 74 to 85 with a mixture of hydrogen and carbon dioxide.
90. The process of statement 89, wherein step a) is conducted at a temperature of 200-380°C.
91. The process of statement 89, wherein step a) is conducted at a temperature of 240-320°C.

### EXAMPLES

One or more examples of the invention will now be described, for the purpose of illustration only, with reference to the accompanying figures:
Figure 1. Powder XRD pattern and single-phase Pawley refinement results of (a) MCM-48@Cu_{1.3} LDH-ori and (b) Cu_{1.3}ZnAl LDH. The observed, calculated, extracted Cu_{1.3}ZnAl LDH phase, background and difference are shown in red, black, blue, yellow and grey, respectively. (hkl) plane reflection of LDH was indicated with short sticks.
Figure 2. FTIR spectra of MCM-48, MCM-48@Cu_{1.3} LDH-ori and Cu_{1.3}ZnAl LDH.
Figure 3. TGA and Derivative TGA (dTG) plots of (a) MCM-48, (b) MCM-48@Cu_{1.3} LDH-ori and (c) Cu_{1.3}ZnAl LDH. The observed TGA, observed dTG and cumulative dTG are plotted with blue, grey, and grey dashed lines. The deconvoluted dTG peaks are coloured in grey, red, blue, yellow and green.
Figure 4. TEM images of (a) MCM-48 (b) zoom-in of MCM-48, (c) Cu_{1.3}ZnAl LDH, (d) MCM-48@Cu_{1.3} LDH-ori and (e) its zoom-in. (f) The schematic illustration of MCM-48@Cu_{1.3} LDH-ori.
Figure 5. (a) ²⁷Al solid-state NMR of MCM-48@Cu_{1.3} LDH-ori and Cu_{1.3}ZnAl LDH. (b) ¹H NMR of MCM-48@Cu_{1.3} LDH-ori.
Figure 6. (a) The N₂ adsorption and desorption isotherm of MCM-48, MCM-48@Cu_{1.3} LDH-ori and Cu_{1.3}ZnAl LDH at 77K. (b) DFT pore size distribution of MCM-48 and LDH at micropore range, (c) DFT pore size distribution of MCM-48 and MCM-48@Cu_{1.3} LDH-ori based on the kernels for metal oxide with cylinder pore and (d) The BJH pore size distribution.
Figure 7. The BET specific surface areas and total pore volume of MCM-48, physical mixture, MCM-48@Cu_{1.3} LDH-ori and Cu_{1.3}ZnAl LDH.
Figure 8. Powder XRD pattern and Rietveld refinement of (a) MCM-48@Cu_{1.3} LDH-67wt and (b) MCM-48@Cu_{1.3} LDH-pH9. The observed, calculated, background, extracted LDH phase, and difference are shown in red, black, mustard yellow, blue (and purple), and grey, respectively. (hkl) reflections of LDH were indicated with short sticks.
Figure 9. TEM images of (a) and (b) MCM-48@Cu_{1.3} LDH-ori, (c) and (d) MCM-48@Cu_{1.3} LDH-67wt, (e) (f) MCM-48@Cu_{1.3} LDH-pH9.
Figure 10. (a) The N₂ isotherm of MCM-48@Cu_{1.3} LDH-ori, -67 and -pH 9 core-shells at 77K. (b) DFT pore size distribution based on the kernels for metal oxide with cylinder pore, (c) The BJH pore size distribution, and (d) BET specific surface area and total pore volume.
Figure 11. Schematic representation of the effect of increasing LDH fraction and reducing the reaction pH on the morphology of core-shells and the corresponding mechanisms.
Figure 12. Powder XRD pattern and Pawley refinement of (a) MCM-48@Cu_{1.3} LDH-pH9-60wt and (b) MCM-48@Cu_{1.3} LDH-pH9-67wt. The observed pattern, cumulative pattern, background, calculated LDH phase, and difference are shown in red, black, mustard yellow, blue (and purple), and grey. (hkl) plane reflection of LDH was indicated with short sticks.
Figure 13. TEM images of (a) and (b) MCM-48@Cu_{1.3} LDH-pH9-50wt, (c) and (d) MCM-48@Cu_{1.3} LDH-pH9-60wt, (e) and (f) MCM-48@Cu_{1.3} LDH-pH9-67wt.
Figure 14. (a) TGA and -dTG, (b) FTIR spectra of MCM-48@Cu_{1.3} LDH-pH9-50wt, 60wt and 67wt.
Figure 15. (a) The N₂ isotherm at 77K and (b) The BJH pore size distribution of MCM-48@Cu_{1.3} LDH-pH9-50wt, -60wt, and -67wt.
Figure 16. (a)The obtained and theoretical BET specific surface area, and (b) The obtained and theoretical total pore volume of MCM-48@Cu_{1.3} LDH-pH9 with varied LDH amount.
Figure 17. XRD pattern of (a) CuₓZnAl LDH, (b) MCM-48@Cuₓ LDH. x = 1.3, 2, 3, 3.5 and 4.
Figure 18. TEM images of (a) Cu_{1.3}ZnAl LDH, (b) Cu₂ZnAl LDH, (c) Cu₃ZnAl LDH, (d) Cu_{3.5}ZnAl LDH, and (e) Cu₄ZnAl LDH
Figure 19. TEM images of (a) MCM-48@Cu_{1.3} LDH, (b) MCM-48@Cu₂ LDH, (c) MCM-48@Cu₃
Figure 20. (a) Electron image of MCM-48@Cu_{3.5}LDH, and TEM-EDX elemental mapping of (b) Cu, (c) Si, (d) Al, and (e) Zn.
Figure 21. The pore size distribution of (a) CuₓZnAl LDH based on the kernels for slit pore, and (b) MCM-48@Cuₓ LDH based on the kernels for metal oxide with cylinder pore.
Figure 22. The BET specific surface area and total pore volume of (a) CuₓZnAl LDH and (b) MCM-48@Cuₓ LDH.
Figure 23. SEM images of (a) ES757, (b) ES757@Cu₄ LDH-Ori. (c) The zoom-in image of the orange-box region in (b). (d) - (g) is the elemental mapping of Si, Cu, Al and Zn, respectively at the orange-box region.
Figure 24. The XRD plots of ES757@Cu4 LDHs from the different synthetic conditions.
Figure 25. High-resolution (HR) SEM image of (a) ES757@Cu₄ LDH-AI, (b) ES757@Cu₄ LDH-Al + conc and (c) ES757@Cu₄ LDH-conc.
Figure 26. (a) The N₂ adsorption and desorption isotherm (at 77 K) of ES757@Cu₄ LDH from different conditions. The insert plots are isotherms of ES757, ES757@Cu₄ LDH-conc and Cu₄ZnAl LDH. (b) The BET specific surface area of ES757, Cu₄ZnAl LDH, their physical mixture with composition (SiO₂:LDH = 40:60) and ES757@Cu₄ LDHs from various conditions.
Figure 27. (a) The total pore volume and (b) the BJH pore size distribution of ES757, physical mixture ES757@Cu₄ LDH from the different synthetic conditions and Cu₄ZnAl LDH.
Figure 28. XRD profiles freshly prepared ES757@Cux LDH.
Figure 29 HR-SEM images showing the shell of (a) ES757@Cu0.8 LDH, (b) ES757@Cu1.3 LDH, (c) ES757@Cu2 LDH, (d) ES757@Cu3 LDH and (e) ES757@Cu4 LDH.
Figure 30 The HR-SEM shows the cross-section region of (a) ES757@Cu3 LDH, (b) ES757@Cu2Figure 31. (a) The BET specific surface area and (b) the total pore volume of ES757@Cux LDH, where x = Cu/AI ratio.
Figure 32. (a) Temperature-programmed reduction profile of ES757@Cux LDO and fresh copper hydroxide carbonate. (b) XRD profiles of ES757@Cux catalyst (hydrogen reduced ES757@Cux LDO at 290 °C for 3 h), peaks marked with star are from the sample holder.
Figure 33. TEM images of (a) ES757@Cu1.3 LDO, (b) ES757@Cu1.3, (d) ES757@Cu2 LDO and (e) ES757@Cu2. HR-SEM images showing the shell of (c) ES757@Cu1.3 and (f) ES757@Cu2.

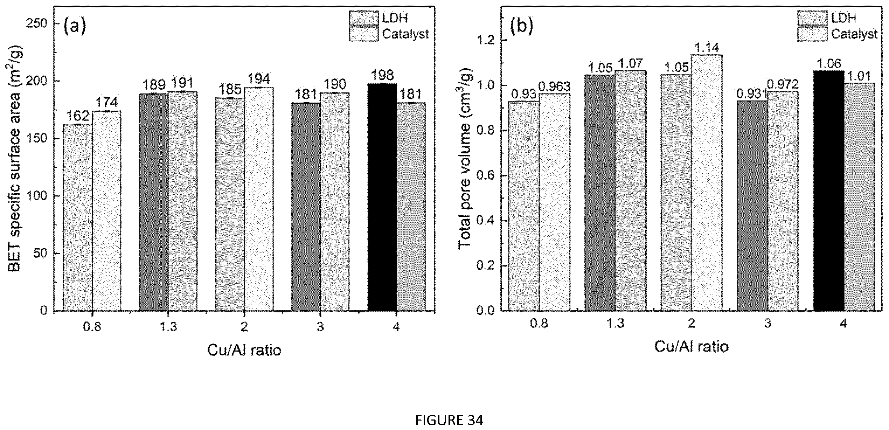

Figure 34. (a) The BET specific surface area and (b) the total pore volume of ES757@Cux LDH and ES757@Cux catalyst, where x = Cu/AI ratio.
Figure 35. (a) CO₂ conversion and (b) space time yield of ES757@Cux LDO. Reaction condition: T = 230-350 °C, P = 45 bar, H₂:CO₂ (molar) = 3:1. WHSV = 18000 mL/g_{cat}/h. The name of catalyst is shortened in the figure for clarity.
Figure 36. The CO₂ conversion, methanol selectivity and STY of ES757@Cux for CO₂ hydrogenation to methanol at 270 °C. The sample name was shorted for clarity.
Figure 37. The CO₂ conversion, methanol selectivity and STY of catalyst derived from Cu1.3ZnAl LDH and ES757@Cu1.3 for CO₂ hydrogenation to methanol at 270 °C.
Figure 38 TEM images of (a) ES757@Cu0.8, (b) ES757@Cu1.3, (c) ES757@Cu2, (d) ES757@Cu3 and (e) ES757@Cu4.

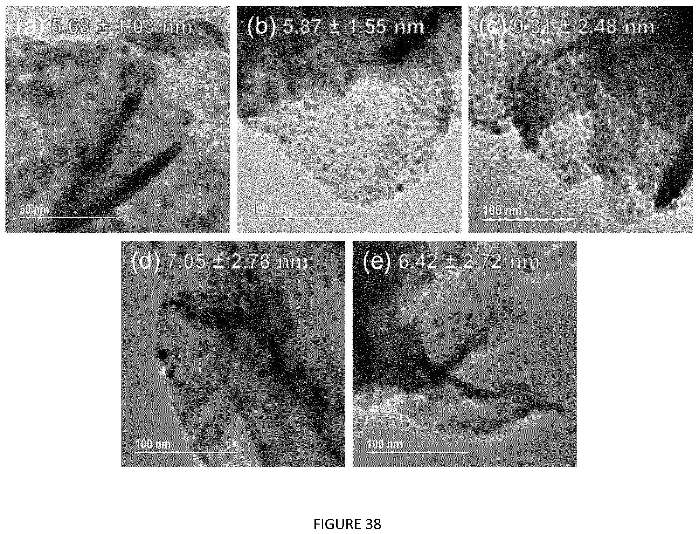

Figure 38. (a) CO₂ conversion and (b) space time yield of ES757@Cux. Reaction condition: T = 230-350 °C, P = 45 bar, H₂:CO₂ (molar) = 3:1. WHSV = 18000 mL/g_{cat}/h. The name of catalyst is shortened in the figure for clarity.

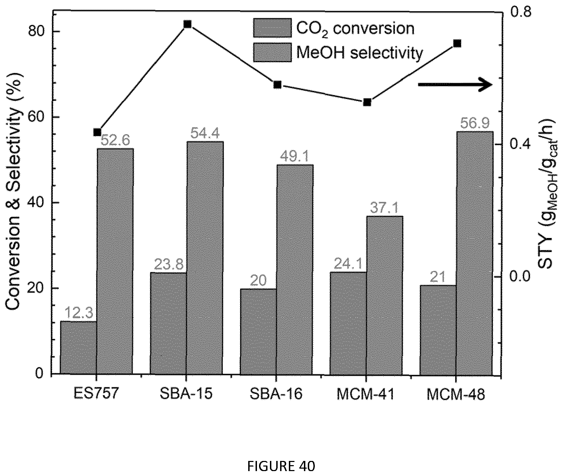

Figure 40. The CO₂ conversion, methanol selectivity and STY of A@Cu2 for CO₂ hydrogenation to methanol at 270 °C. The sample name was shorted to the name of their mother silica for clarity.

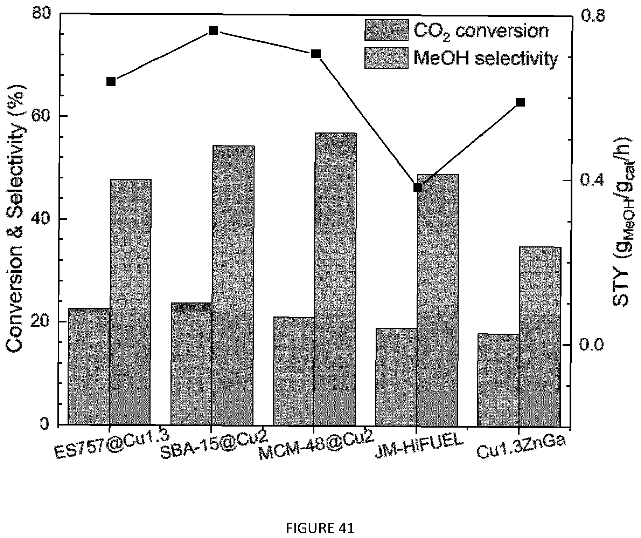

Figure 41. Comparison of CO₂ conversion, selectivity, and methanol yield of ES757@Cu1.3. SBA-15@Cu2, MCM-48@Cu2, and an industrial sample, HiFUEL for the CO₂ hydrogenation to methanol at 270 °C, 45 bar with a H2/CO2 feed (H₂:CO₂ = 3:1). The catalytic performance of JM-HiFuel and Cu1.3ZnGa is obtained from the literature.³³

### 1.1 Synthesis of MCM-48@CuₓZnAl LDH

### 1.1.1. Synthesis and characterisation of MCM-48@CuₓZnAl LDH

Core-shell MCM-48@Cu_{1.3}ZnAl-CO₃-LDH was prepared using the co-precipitation method. The LDH platelets grow *in-situ* on the surface of MCM-48 silica. An aqueous solution of copper, zinc and aluminium nitrate, with Cu:Zn: Al molar ratio = 1.3:1:1, was added to an aqueous dispersion of MCM-48 in Na₂CO_{3(aq)} at pH 10. The pH of the reaction mixture was maintained at 10 using 1 M NaOH_{(aq)} solution. After completing the addition of metal nitration, the resulting mixture was aged under stirring for 1 hour. The solid product was collected by filtration and washed with DI water before drying under vacuum at 30 °C overnight. The product is a blue powder named MCM-48@Cu_{1.3} LDH-ori. The pristine CuZnAl LDH was prepared with the same conditions as MCM-48@Cu_{1.3} LDH-ori without MCM-48 core and was termed Cu_{1.3}ZnAl LDH.

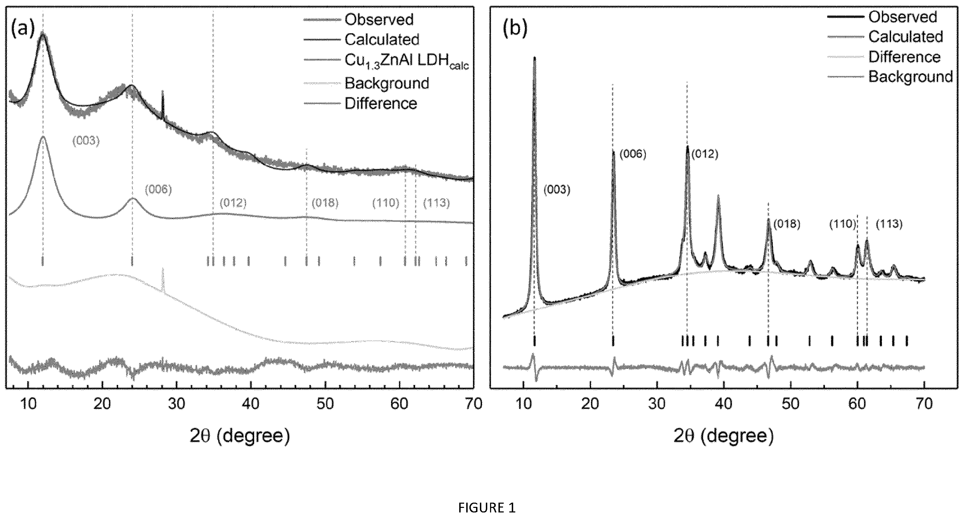

The MCM-48 used in this study is a commercial silica with highly-ordered mesopores which gives characteristic powder X-Ray diffraction (XRD) pattern at 2θ lower than 3° and an amorphous peak at 2θ around 24°.¹ MCM-48@Cu_{1.3} LDH-ori and Cu_{1.3}ZnAl LDH exhibit typical LDH reflections (Figure 1). No phase associated with impurities is observed.

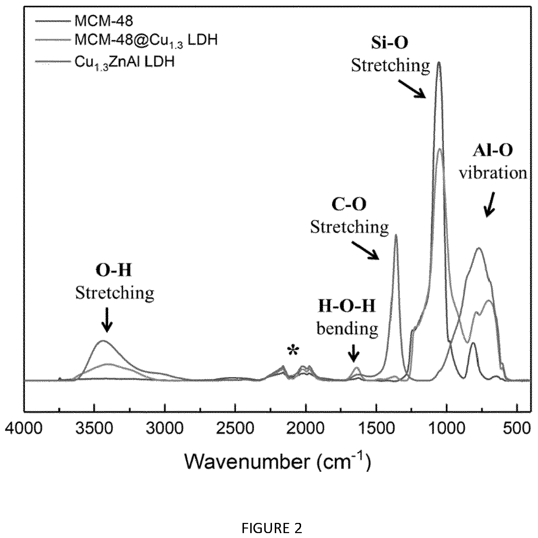

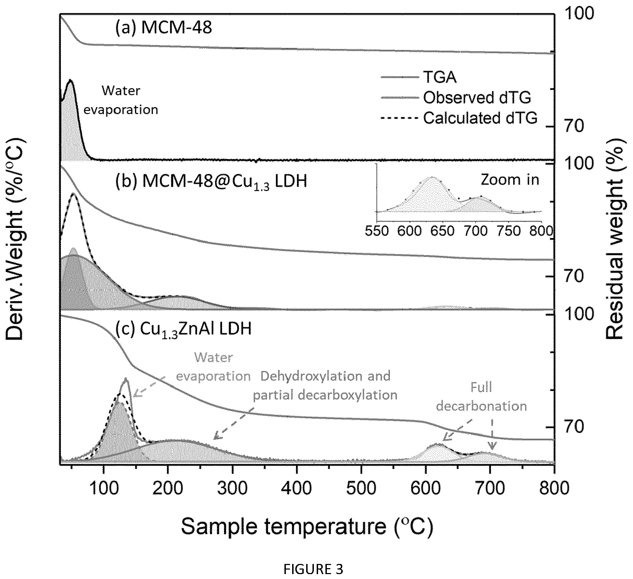

The FTIR spectra of MCM-48, MCM-48@Cu_{1.3} LDH-ori and Cu_{1.3}ZnAl LDH are shown in Figure 2. The thermostability of materials was studied with TGA (Figure 3).

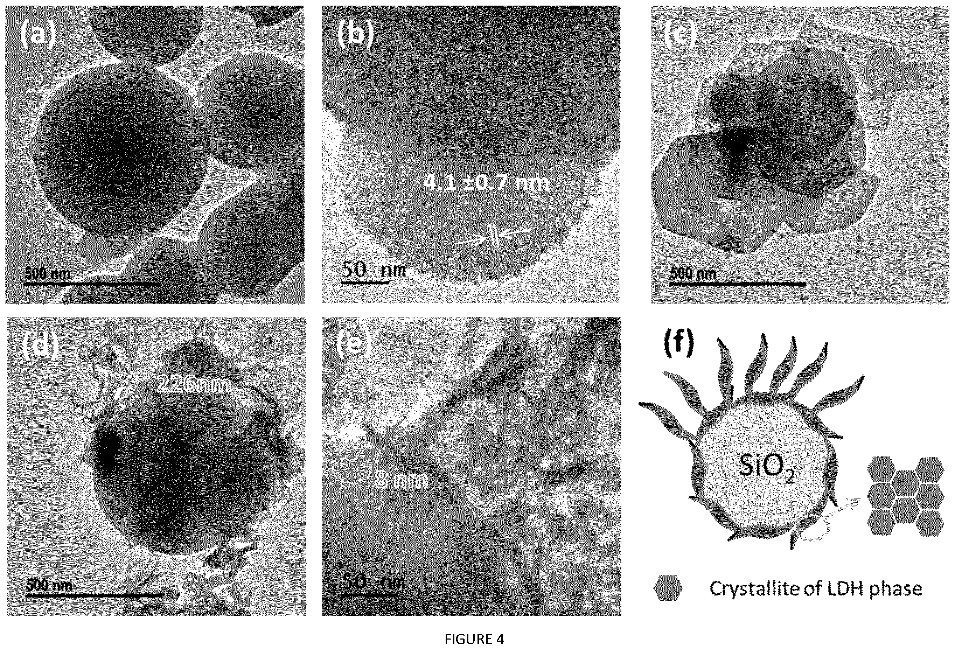

MCM-48 are spherical particles with a diameter of around 604 ± 159 nm (Figure 4a), the pore size is about 4.1 ± 0.7 nm as revealed by the TEM image (Figure 4b). Cu_{1.3}ZnAl LDH is the aggregation of hexagonal platelets with a diameter between 175 and 500 nm (Figure 4c). MCM-48@Cu_{1.3} LDH-ori displays a typical core-shell morphology with a spherical core and a seaweed-like shell of which the thickness varies from 8 nm to 226 nm (Figure 4d and e). A schematic illustration of the structure of MCM-48@Cu_{1.3} LDH-ori is given in Figure 4f based on the observed morphology. It is proposed that small crystallites of LDH firstly wrap on the surface of silica, then radially grow from the interface to give non-rigid seaweed-like shells.

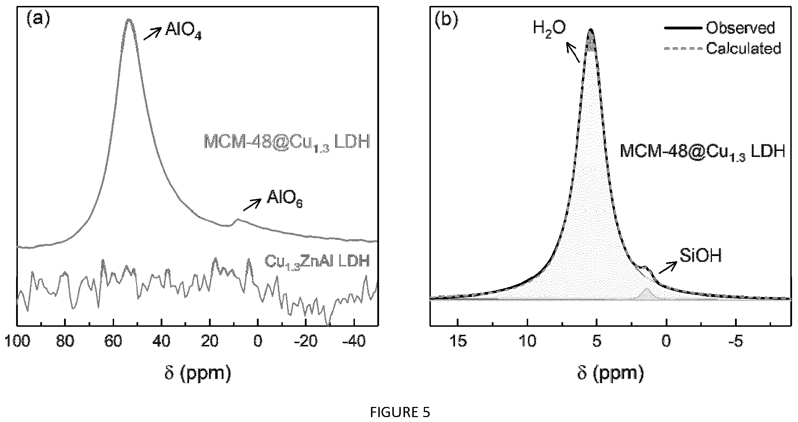

Figure 5a shows the ²⁷Al solid-state NMR of the core-shell and LDH. Al³⁺ is octahedrally coordinated in Cu_{1.3}ZnAl LDH and is expected to give a band at around 10 ppm.^{22,23} However, the neighbouring paramagnetic Cu²⁺ leads to an expansion in chemical shift and a band broadening, which makes the 10 ppm resonance unobservable.^{24,25} Two Al resonances are seen in core-shell. The 53.3 ppm band is assigned to tetrahedrally coordinated Al (AlO₄).²⁶ Si atoms are 4-coordinated in MCM-48.²⁷ The presence of AlO₄ is very likely due to the formation of aluminosilicate at the interface.²⁷ The other 8.2 ppm signal is associated with the octahedral Al (AlO₆).^{22,23}.

The ¹H NMR spectrum of the composites contains two features: one dominant peak at 5.4 ppm due to the interlayer water and 1.4 ppm resulting from the silanol group, SiOH.^{28,29}

The pore structure is investigated by the N₂ adsorption and desorption isotherm at 77K. Surface area is estimated using the Brauner-Emmett-Teller (BET) method. Pore size distribution within the micropore (< 2nm) and mesopore (2-50 nm) is calculated by applying a DFT kernel on the adsorption isotherm. For silica and core-shell composite, the adsorption of N₂ at 77 K on metal oxide with cylindrical pore kernel was selected. For LDH, the adsorption of N₂ at 77 K with slit pore kernel was adopted. Macropore (> 50nm) size distribution is determined utilising Barrett-Joyner-Halenda (BJH) method.

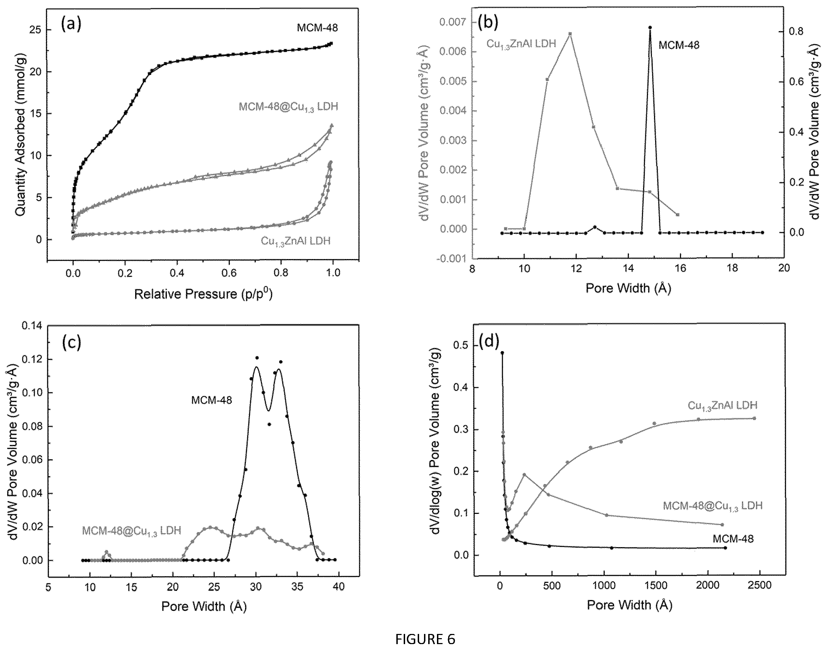

MCM-48 that shows type IVb isotherm with no hysteresis loop is a mesoporous material with a pore size smaller than 4 nm (Figure 6a).³⁰ DFT pore size distribution further confirmed that MCM-48 shows a narrow pore size distribution near diameter 3.0-3.3 nm and 1.5 nm (Figure 6b and c). Cu_{1.3}ZnAl LDH that exhibits a type II isotherm with H3 hysteresis loop is supposed to be a non-rigid aggregate of plate-like particles that gives slit-like pores.^{30,31} This is consistent with the TEM images. Micropore (1.2 nm from the DFT method) is detected in the LDH (Figure 6b), either due to the micropore defects or aggregation of small LDH crystallites.³² No clear maxima of the pore size distribution can be noticed in the mesopore and macropore range (Figure 6d). It is presumably attributed to the absence of interparticle channels when the LDH flakes are intensely stacked.³³

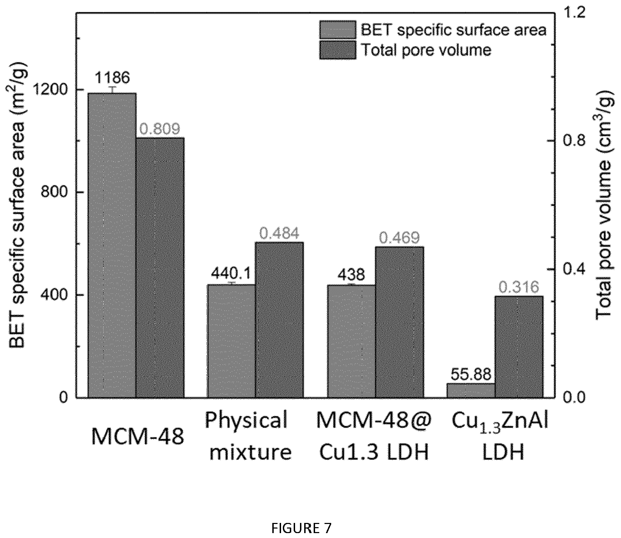

BET surface area and pore volume data are presented in Figure 7.

### 1.1.2. Effect of synthetic condition

The effect of factors such as the LDH fraction, the reaction pH and the metal nitrate dropping rate on the shell morphology were studied. All core-shells were prepared via the co-precipitation method described hereinbefore. Only one factor was changed each time. A summary of the synthetic condition and their relevant samples are given in Table 1.

**Table 1. A summary of the varied synthetic condition, their relevant samples, the expected and the real LDH fraction in the composite.**

| Sample name | Reaction pH | Metal nitrate dropping rate (mmol/min) | LDH fraction (expected) | LDH fraction (real)^{∗} |
|---|---|---|---|---|
| MCM-48@Cu_{1.3} LDH-ori | 10 | 0.05 | 50 | 66 |
| MCM-48@Cu_{1.3} **LDH-67wt** | 10 | 0.05 | **67** | 70 |
| MCM-48@Cu_{1.3} **LDH-pH9** | **9** | 0.05 | 50 | 55 |
| MCM-48@Cu_{1.3} **LDH-0.1** | 10 | **0.1** | 50 | n/a |

| | | | | |
|---|---|---|---|---|
| ^{∗}: the real LDH fraction in the composite is calculated from the TGA results. | | | | |

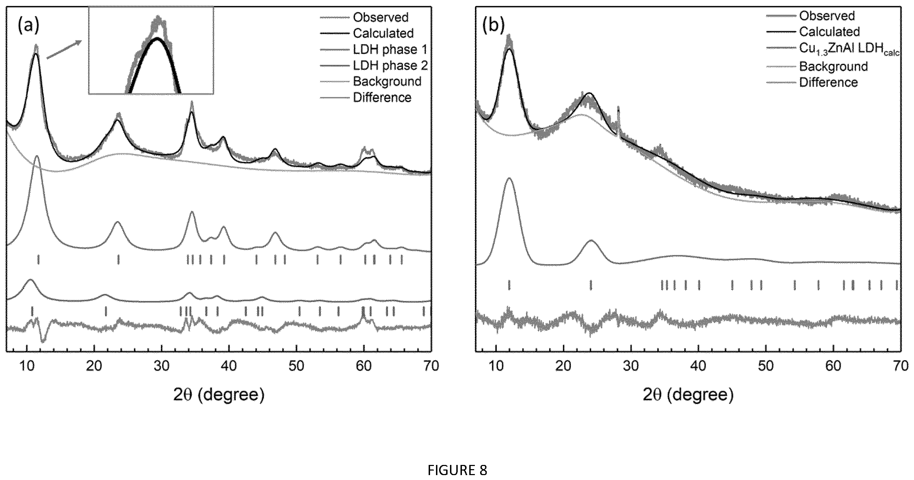

Samples with more LDH and prepared at lower pH both exhibit typical LDH reflections (Figure 8).

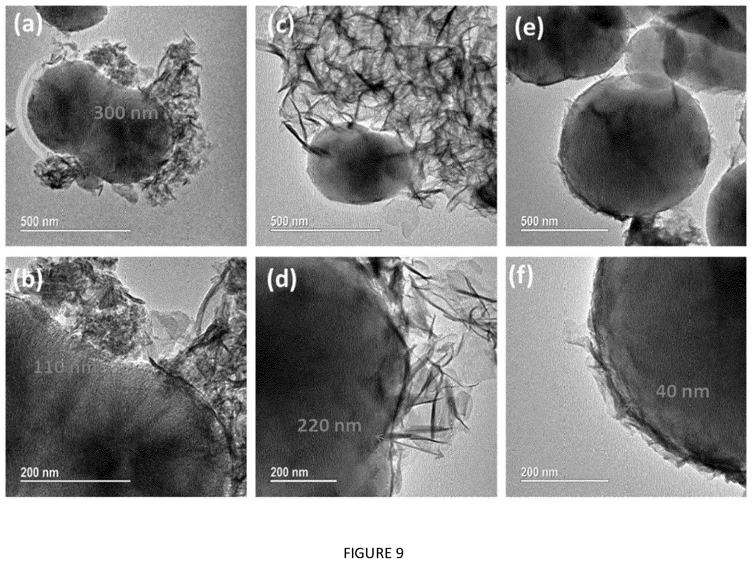

Introducing more LDH in the composite appears to give a better-defined platelet-like morphology in the shell (Figure 9a-d).

Lowering the pH to 9 gave rise to an excellent shell that is thin and uniform, the thickness of which is 40 nm (Figure 9e and f). Moreover, no evident free LDH is observed.

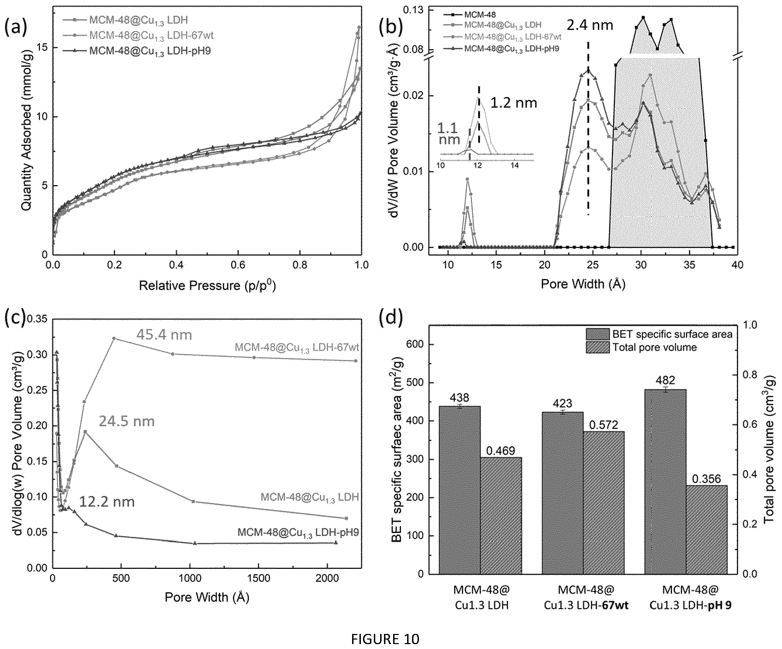

All three core-shells possesses a type IVb isotherm at a low relative pressure (p/p0 ≤ 0.4) due to their mesoporous core (Figure 10a). Composite prepared at pH 9 possesses an almost flat N₂ adsorption curve at high relative pressure accompanied with a H4 loop. This indicates a limited amount of inter-particles void in this material and suggests a thin shell of this composite.³⁰

DFT and BJH pore size distribution reveals that MCM-48@Cu_{1.3} LDH-pH9 has 1.1 nm micropores and mesopores at 2.4 - 3.0 nm and 12.2 nm (Figure 10b and c)

Samples prepared at pH 10 exhibit evident N₂ adsorption at high relative pressure due to the condensation in the inter-particles void and a H3 hysteresis loop. ^{30,34} Enlarged LDH content (67 wt%) gives steeper N₂ adsorption and draw the shape of isotherm closer to that of Cu_{1.3}ZnAl LDH. Additionally, the BJH distribution between pristine LDH and MCM-48@Cu_{1.3} LDH-67wt shows a high similarity between each other, where a wide pore size distribution over meso- and macro-pore region without a clear peak value is observed (Figure 10c and Figure 6d). This observation, along with the TEM image (Figure 9c), suggests the occurrence of some free LDH in MCM-48@Cu_{1.3} LDH-67wt.

The BET specific surface area and total pore volume are shown in Figure 10d. MCM-48@Cu_{1.3} LDH-67wt has a similar surface area (423 m²/g) as MCM-48@Cu_{1.3} LDH-ori (438 m²/g) a higher total pore volume. MCM-48@Cu_{1.3} LDH-pH9 exhibits the highest BET specific surface area (482 m²/g) but the lowest total pore volume (0.356 cm³/g).

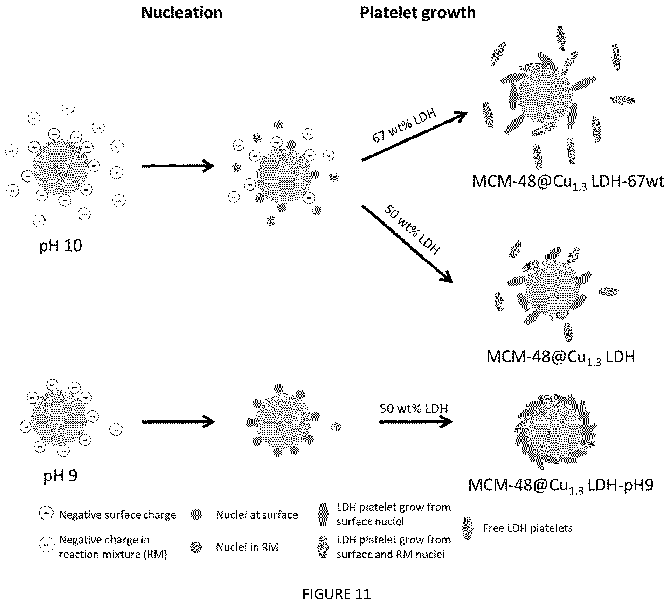

Based on the characterisation results, a schematic illustration about the effect of synthetic condition on the morphology of core-shells is given (Figure 11).

### 1.1.3. Preparing core-shells with varied Cu content

Following the success of core-shell generation, further tuning of the composition to ensure it would have a promising catalytic performance was carried out. Many of the reported Cu/ZnO/Al₂O₃ catalysts for methanol synthesis exhibits a Cu loading over 18wt % (Table 2). It is also noticed that high Cu loading (between 30-50wt %) seems to be related to good catalytic performance (Entry 2-4, Table 2).

**Table 2. Cu loading and the molar ratio of Cu/ZnO/Al₂O₃ methanol synthesis catalyst reported in the literature.**

| Entry | Cu loading (wt %) | Molar ratio | | | Precursor | STY^{a} | Ref |
|---|---|---|---|---|---|---|---|
| | | Cu | Zn | Al | | | |
| 1 | 18.5^{b} | 3 | 1 | n/a | LDH | N/A | 57 |
| 2 | 32.7 | 1.9 | 1.4 | 1^{c} | LDH | 0.59 | 58 |
| 3 | 47.3 | 1.6 | 0.7 | 1 | Oxides | 1.41 | 59 |
| 4 | 51.2 | 8.5 | 3.2 | 1 | Oxides | 0.85 | 60 |
| 5 | 58.6-63.5^{d} | 3.8-8.4 | 1.2-1.4 | 1 | Oxides | N/A | 61 |

| | | | | | | | |
|---|---|---|---|---|---|---|---|
| a: space time yield (g_{MeOH}/g_{cat}/h) b: value was calculated from the local EDX measurement of catalyst. c: Instead of Al, Ga was used in that study, d: calculated from the molar ratio provided. | | | | | | | |

The theoretical Cu content in the catalyst derived from MCM-48@Cu_{1.3} LDH-pH9 is only 12.3 wt % (Table 3). There exist two options for increasing the amount of Cu in the core-shell. One is to increase the LDH fraction; by elevating the quantity of LDH from 50wt % to 67wt %, 16.5wt % Cu loading can be achieved. The other is to increase the Cu density in the LDH enables a further improvement of the Cu population. It is expected that a 25.5wt % Cu content in the catalyst can be obtained from a core-shell precursor where LDH shell occupies 60% weight, and its composition is Cu:Zn: Al = 4:1:1 by molar.

**Table 3. The expected Cu loading of core-shells with different LDH fractions (as indicated in samples name) or different Cu density in LDH (with LDH fraction being kept at 60wt %). Values are calculated from the theoretical formula of catalysts.**

| Samples | Expected Cu loading (wt %) | The molar ratio in LDH | | |
|---|---|---|---|---|
| | | Cu | Zn | Al |
| MCM-48@Cu_{1.3} LDH-pH9**(-50wt %)** | 12.3 | 1.3 | 1 | 1 |
| MCM-48@Cu_{1.3} LDH-pH9-**60wt %** | 14.8 | 1.3 | 1 | 1 |
| MCM-48@Cu_{1.3} LDH-pH9-**67wt %** | 16.5 | 1.3 | 1 | 1 |
| MCM-48@Cu₂ LDH | 18.9 | 2 | 1 | 1 |
| MCM-48@Cu₃ LDH | 22.8 | 3 | 1 | 1 |
| MCM-48@Cu_{3.5} LDH | 24.2 | 3.5 | 1 | 1 |
| MCM-48@Cu₄ LDH | 25.5 | 4 | 1 | 1 |

### 1.1.3.1. Modify LDH content

In order to increase the LDH fraction in the composite, the total amount of the metal nitrate solution was raised; the concentration of nitrate solution and its dropping rate is preserved to minimise the variation of the LDH nucleation. Products are named after their reaction pH and LDH content. From now on, MCM-48@Cu_{1.3} LDH-pH9 will be called MCM-48@Cu_{1.3} LDH-pH9-50wt.

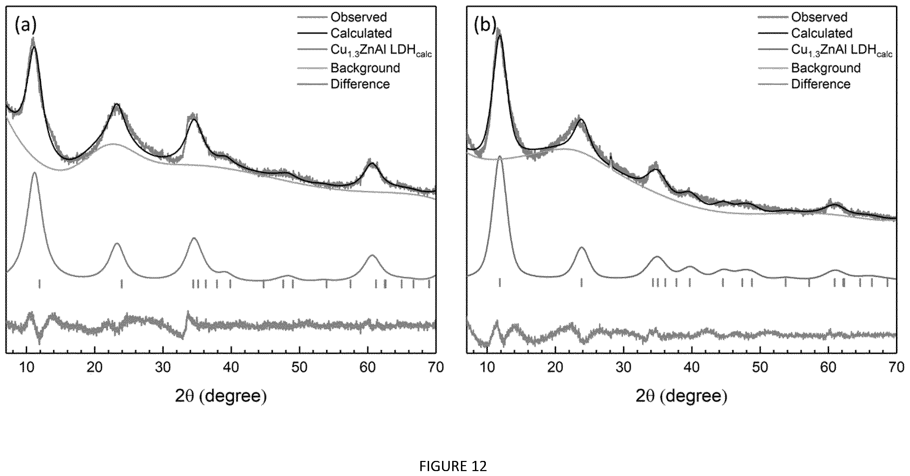

Figure 12a and b show the powder X-ray diffractograms of MCM-48@Cu_{1.3} LDH-pH9-60wt % and 67wt %, respectively. In both samples, the characteristic LDH Bragg reflections are present.

Pawley refinement based on the space group of LDH is conducted on the XRD pattern of three samples. The d-spacing and crystallite size are similar regardless of the LDH content in the composite (Table 4).

**Table 4. The expected and actual LDH content in the composite, as well as the d-spacing and crystallite size of the LDH phase obtained from the Pawley refinement.**

| Sample | LDH fraction (expected) | LDH fraction (real)^{∗} | *d*₀₀₃ (Å) | *d*₁₁₀ (Å) | D₀₀₁ (nm) | D₁₁₀ (nm) |
|---|---|---|---|---|---|---|
| pH9-50 wt | 50 | 55 | 7.39 | 1.50 | 2.54 | 0.94 |
| pH9-60 wt | 60 | 62 | 7.43 | 1.52 | 2.07 | 2.70 |
| pH9-67 wt | 67 | 75 | 7.46 | 1.52 | 2.69 | 2.15 |

| | | | | | | |
|---|---|---|---|---|---|---|
| ^{∗}: Values were attained from the TGA results. | | | | | | |

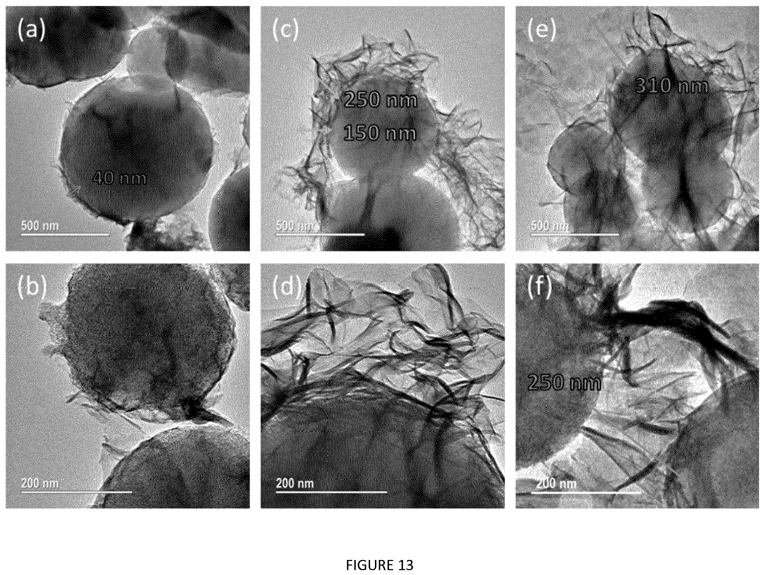

The thickness of the LDH shell shows an upward trend with the increase in the LDH amount, 40 nm for 50wt %, 150-250 nm for 60wt % and 250-350 nm for 67wt % (Figure 13).

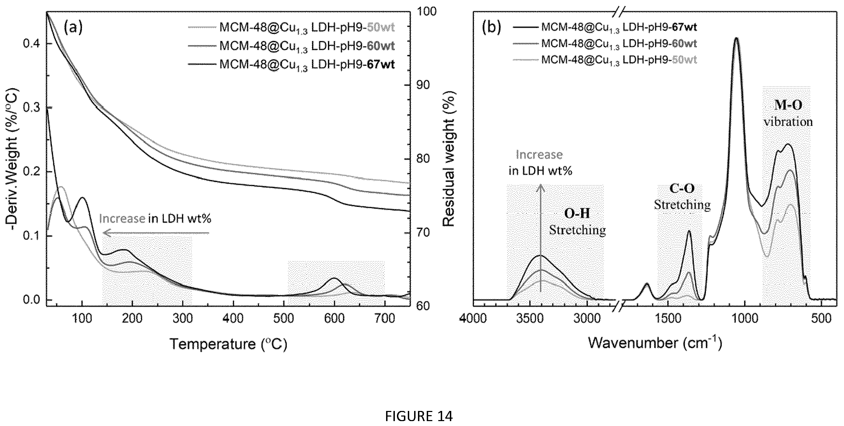

TGA of all samples exhibits distinct LDH weight loss stages (Figure 14a).

The band intensity in FTIR spectra was normalised to the most intense band, Si-O stretching band at ca 1000 cm⁻¹ (Figure 14b). Peaks related to the LDH, including the M-O vibration, C-O stretching and O-H stretching, are enhanced with the raised LDH amount in the composite. This is in line with expectations.

All three samples possess a typical type IV isotherm at low p/p0 due to the presence of micro and mesopore. The DFT pore size distribution illustrates that these composites exhibit similar pore size distribution at pore width below 4 nm, where 1.2 nm and 2.4-3.0 nm pores are detected.

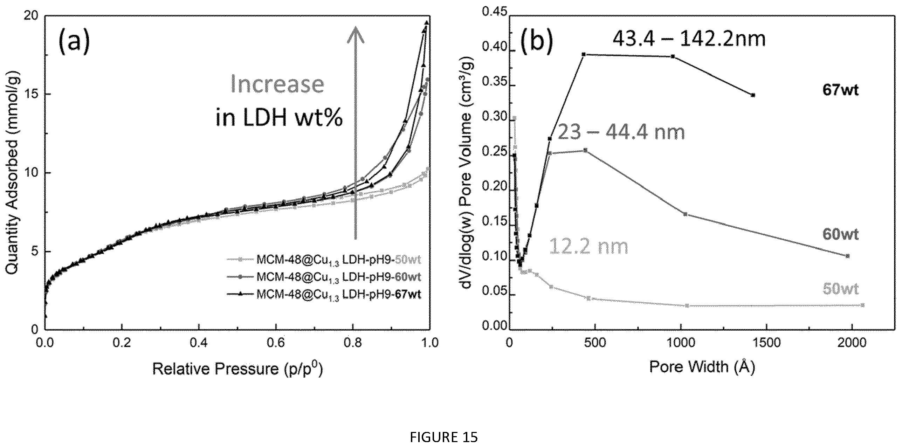

At higher relative pressure, N₂ adsorption increases with the increase in LDH content (Figure 15a). Adsorption at this region originates from the N₂ condensation in the interparticle voids due to particle-particle, particle-platelet, platelet-platelet aggregation.^{30,34}

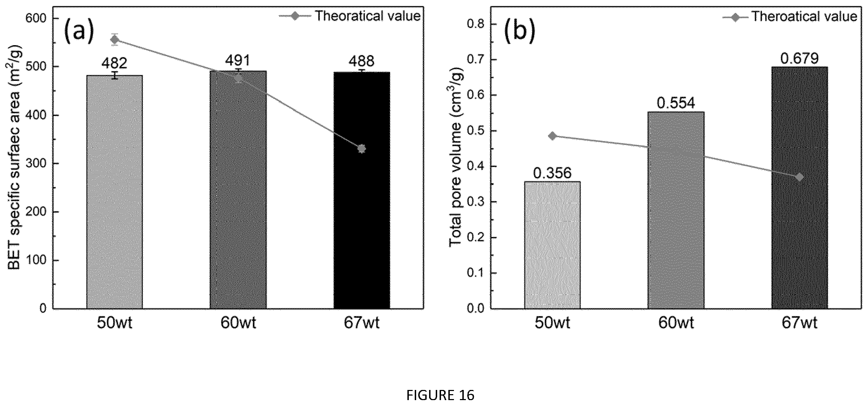

The BET specific surface area is almost identical among the three samples (Figure 16a).

### 1.1.3.2. Modify Cu density in LDH

To further increase the Cu amount in the core-shell, the Cu/AI molar ratio was increased. The LDH fraction in the composite is fixed at 60wt % to enable a moderate amount of Cu amount, well-defined shell and a low level of free LDH. The total metal nitrate amount involved in the synthesis was kept constant while the metal ratio was varied. It is known that pure LDH can only form at M^{II}/M^{III} ratio between 1.5-5.²¹ Setting the Zn/AI ratio to 1 in all cases, the Cu/AI molar ratio changes from 1.3 to 4. Products are named as MCM-48@Cuₓ LDH, where x is the Cu/AI molar ratio. From now on, MCM-48@Cu_{1.3} LDH-pH9-60wt % is called MCM-48@Cu_{1.3} LDH. Pure LDHs are prepared under the same synthetic condition without MCM-48 and are termed CuₓZnAl LDH.

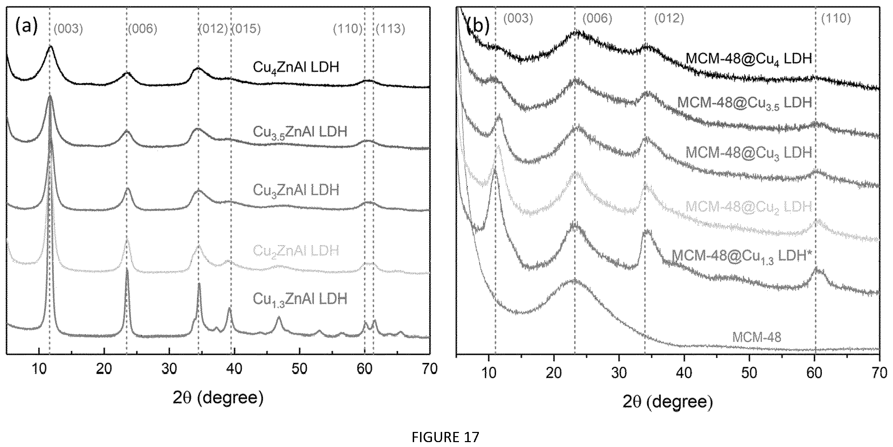

Figure 17 shows the XRD diffractograms of CuₓZnAl LDH and MCM48@Cuₓ LDH, x = 1.3, 2, 3, 3.5, and 4. All samples exhibit the characteristic LDH pattern without any reflection from impurities. It is observed that LDH is formed in all cases and is the only crystalline phase in the sample.

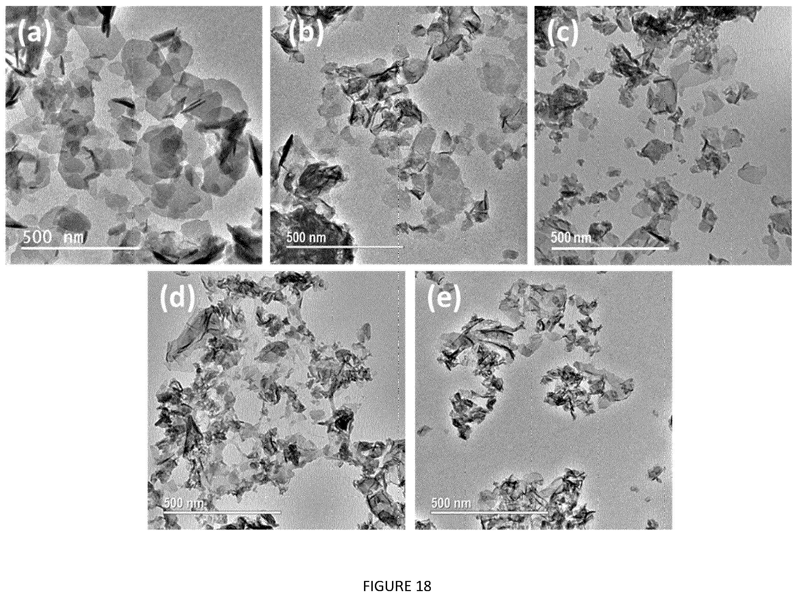

Figure 18 show the TEM images of LDHs. It is seen that LDHs possess platelet-like morphology irrespective of the Cu density. However, the particle size decreases with the raised copper population, where small fragments with irregular shapes are more common at high Cu loading than large hexagonal sheets.

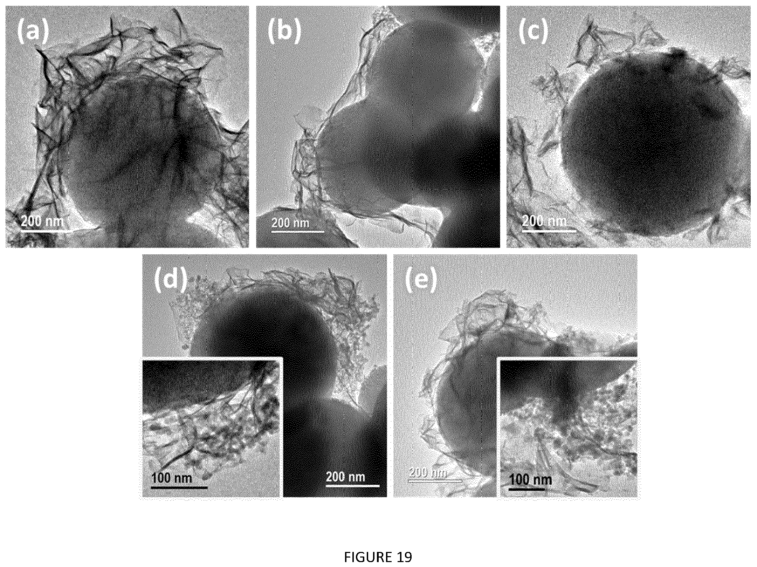

Core-shell morphology is obtained in all composites (Figure 19). There is no clear trend between the thickness of the shell and the Cu density.

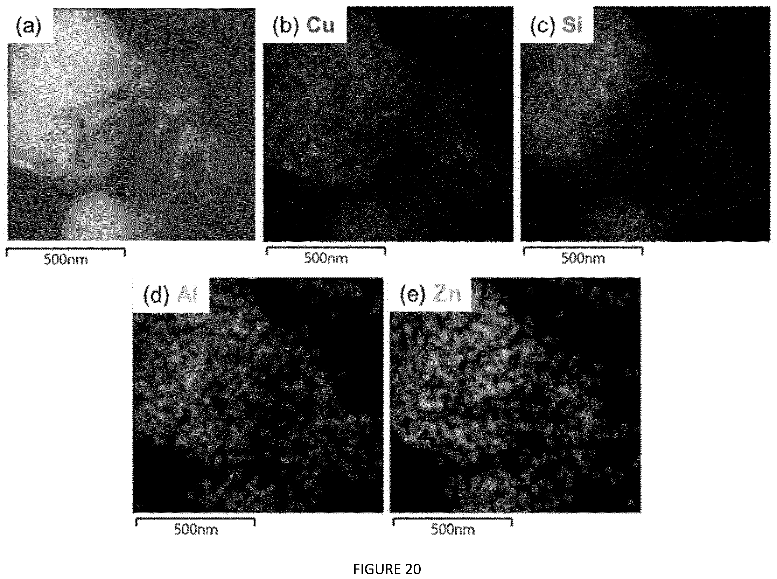

Figure 20 shows the elemental mapping of MCM-48@Cu_{3.5} LDH. A uniform distribution of elements over particles has been achieved.

The Cu/AI ratios determined by ICP-OES agrees with the theoretical values in both cases of pristine LDHs and composites (=Tables 5 and 6).

**Table 5. The expected and experimental Cu: Al molar ratio in CuₓZnAl LDHs with their possible chemical formula and impurity.**

| Cu/Al ratio | | Stoichiometry in LDH [CuₐZn_{b}Al_{c}(OH)_{2(a+b+c)}(CO₃)_{c/2}] nH₂O | | | | | | Impurity Cu₂CO₃(OH)₂^{b} |
|---|---|---|---|---|---|---|---|---|
| Expected^{a} | Real | Cu | Zn | Al | OH | CO₃²⁻ | H₂O | |
| 1.3 | 1.31 | 1.10 | 0.93 | 1 | 6.28 | 0.5 | 4 | 0.106 |
| 2 | 1.94 | 1.29 | 0.93 | 1 | 7.09 | 0.5 | 4 | 0.323 |
| 3 | 2.93 | 2.18 | 0.90 | 1 | 8.92 | 0.5 | 1.6 | 0.373 |
| 3.5 | 3.47 | 2.68 | 0.94 | 1 | 10.0 | 0.5 | 3.3 | 0.394 |
| 4 | 4.06 | 2.84 | 0.92 | 1 | 10.7 | 0.5 | 1.6 | 0.612 |

| | | | | | | | | |
|---|---|---|---|---|---|---|---|---|
| a: expected Cu/Al ratio based on the amount of starting material in the co-precipitation. b: Impurity could be a mixture of zinc hydroxide carbonate and copper hydroxide carbonate. | | | | | | | | |

The expected chemical formula of MCM-48@Cuₓ LDHs is listed in Table 6.

**Table 6. The expected and experimental Cu: Al molar ratio in MCM-48@Cuₓ LDHs with their possible chemical formula and impurity.**

| Cu/Al ratio | | Stoichiometry in LDH [CuₐZn_{b}Al_{c}(OH)_{2(a+b+c)}(CO₃)_{c/2}] nH₂O | | | | | | SiO₂ | | Cu₂CO₃ (OH)₂^{b} |
|---|---|---|---|---|---|---|---|---|---|---|
| Exp^{a} | Real | Cu | Zn | Al | OH | CO₃²⁻ | H₂O | NaAlO₂ | SiO₂ | |
| 1.3 | 1.37 | 1.37 | 0.96 | 0.77 | 6.2 | 0.39 | 1.5 | 0.23 | 3.5 | 0 |
| 2 | 2.04 | 2.04 | 0.92 | 0.89 | 7.7 | 0.45 | 1.0 | 0.11 | 4.4 | 0 |
| 2 | 2.04 | 1.94 | 0.92 | 0.8 | 7.3 | 0.4 | 1.3 | 0.2 | 4.4 | 0.04 |
| 3 | 3.04 | 2.91 | 0.92 | 0.9 | 9.5 | 0.45 | 1.9 | 0.1 | 5.5 | 0.06 |
| 3 | 3.04 | 2.81 | 0.92 | 0.8 | 9.1 | 0.4 | 2.1 | 0.2 | 5.5 | 0.12 |
| 3.5 | 3.38 | 3.07 | 0.96 | 0.8 | 9.6 | 0.40 | 1.8 | 0.2 | 6 | 0.15 |
| 4 | 4.04 | 3.05 | 0.98 | 0.8 | 9.7 | 0.40 | 1.8 | 0.2 | 6.5 | 0.49 |

| | | | | | | | | | | |
|---|---|---|---|---|---|---|---|---|---|---|
| a: expected Cu/Al ratio based on the amount of starting material of co-precipitation. b: Impurity could be a mixture of zinc hydroxide carbonate and copper hydroxide carbonate. | | | | | | | | | | |

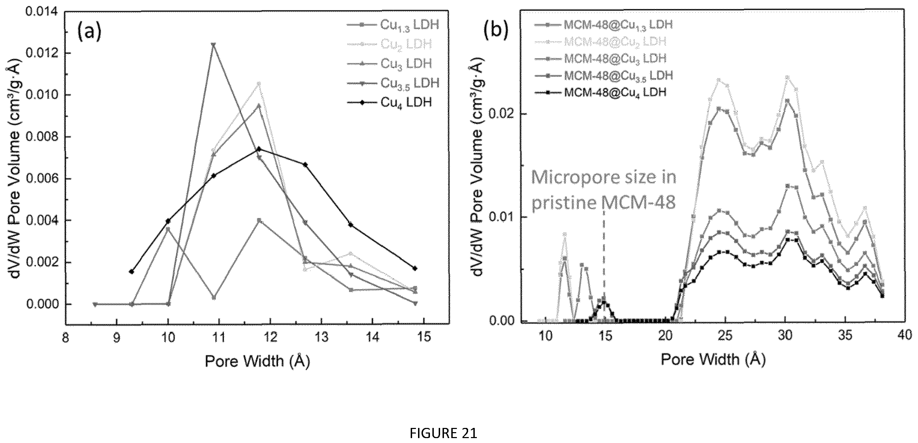

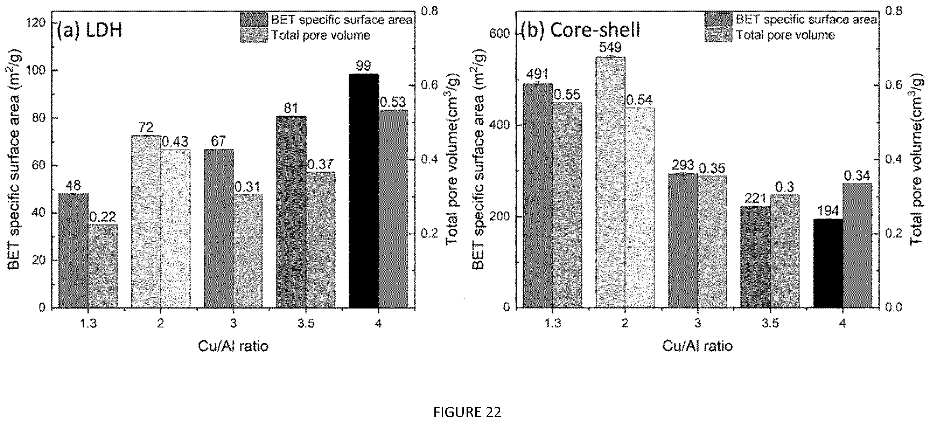

Pore size distribution data are shown in Figure 21. BET surface area and pore volume data are shown in Figure 22.

### 1.2. Synthesis of SiO₂@CuₓZnAl LDH with commercial silica ES757

ES757, a commercial silica, was used to develop SiO₂@CuZnAl LDH core shell-derived catalysts due to its scalable synthesis and industrial application.

### 1.2.1. Effect of synthetic condition on ES757@Cu₄ LDH

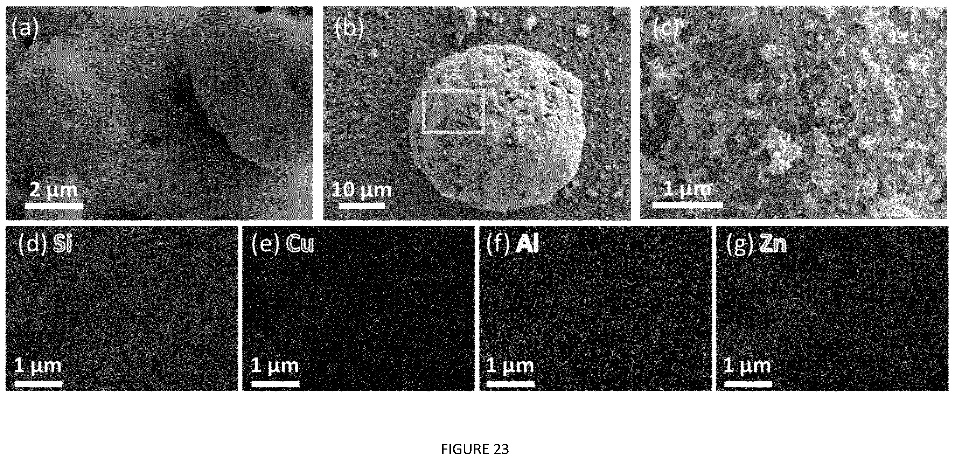

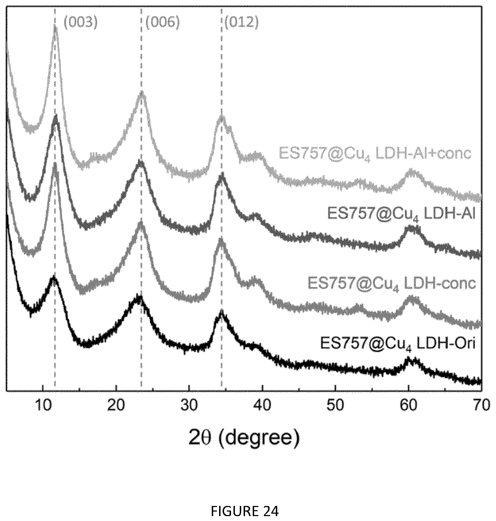

ES757-based core-shell was firstly prepared with the highest copper loading. ES757@Cu₄ LDH-Ori was obtained using the same condition as MCM-48@Cu₄ LDH. To achieve enough sample for characterisation and catalyst evaluation from one batch, synthesis was attempted at an eight-times scale. A clean XRD pattern that only shows the typical LDH reflections proves the product's purity (Figure 24). ES757 are bulky spherical silica with a smooth surface (Figure 23a). SEM image and EDX mapping of ES757@Cu₄ LDH-Ori reveals that the LDH shell has formed on the surface of ES757 (Figure 23b-g).

Previous work has shown that using aluminium impregnation and concentrated reaction mixture can improve the LDH coverage and reducing free LDH amount in the large-scale SiO₂@LDH preparation.^{36,37}

In Al impregnation, silica was pre-treated. Al atoms decorate SiO₂ via wet impregnation. Those Al atoms would act as the nucleation site of LDH and facilitate the growth of LDH on the surface of SiO₂. In the concentrated reaction mixture, the LDH nuclei have a higher chance of contact with silica core, which would boost the formation of supported nuclei and gain better LDH coverage and a lower level of free LDH.

To eliminate the free LDH, Al impregnation, concentrated condition, and a combination of both were attempted. The sample prepared from the Al-impregnated silica is called ES757@Cu₄ LDH-AI. For concentrated conditions, the amount of metal nitrate and silica involved in the synthesis is maintained the same. The amount of DI water used to get the metal nitrate solution is reduced to 38% of the original amount; for the silica dispersion, 15% of water was used. Accordingly, the metal nitrate dropping rate was adjusted to ensure metal cations were added at the same speed in both the original and concentrated conditions. The sample prepared from the concentrated conditions is termed ES757@Cu₄ LDH-conc. The sample obtained from the Al-impregnated silica under concentrated conditions is named ES757@Cu₄ LDH-AI + conc.

The XRD pattern is unchanged (Figure 24). LDH is the only crystalline phase observed in all samples.

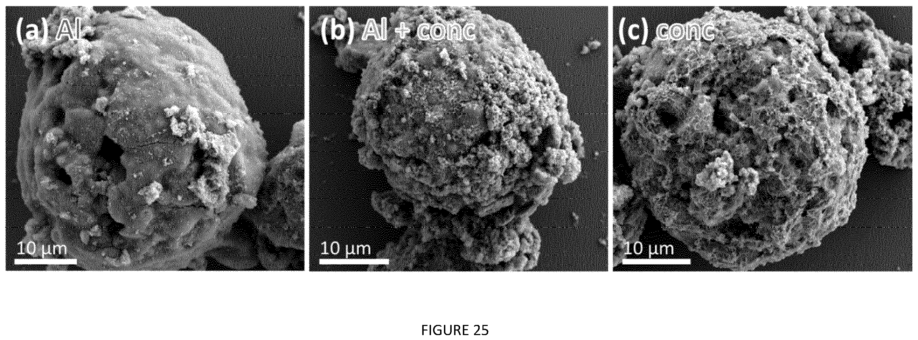

High-resolution (HR) SEM images are shown in Figure 25. A clean background is obtained when one of or both the Al impregnation and concentrated condition is applied. This indicates a successful elimination of any free LDH.

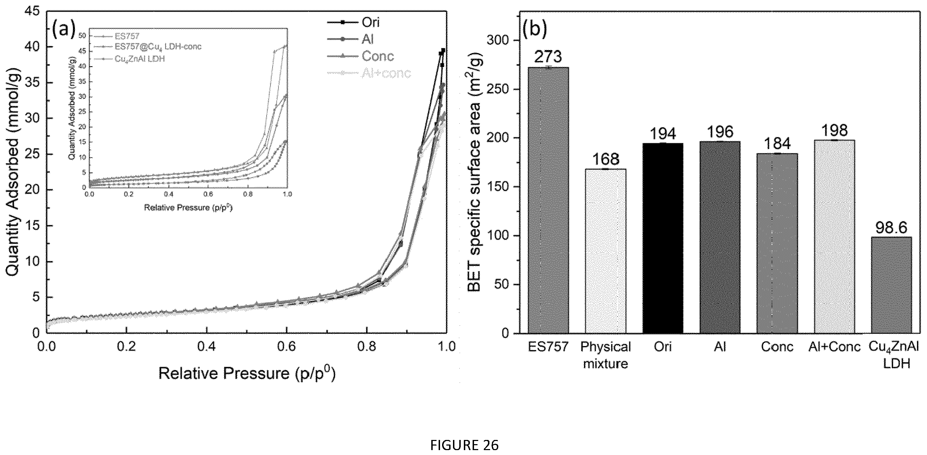

The BET specific surface area is almost identical in all core-shells and is higher than that of the physical mixture (Figure 26b).

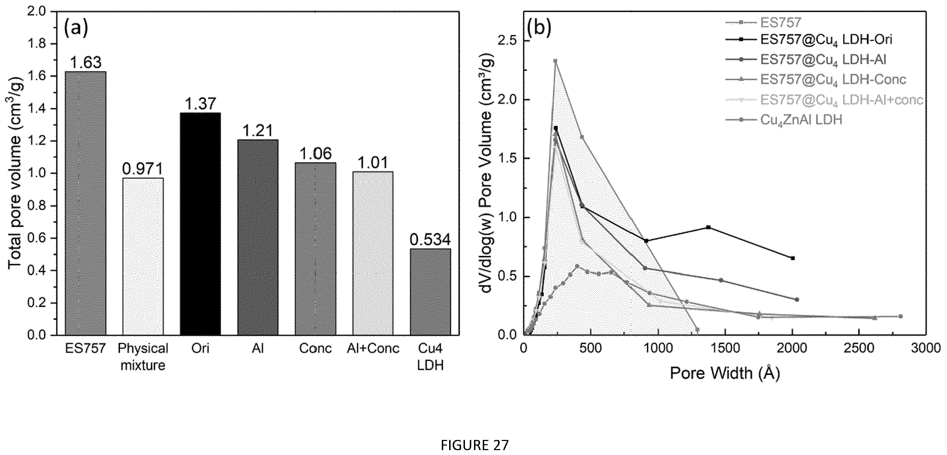

The total pore volume of core-shells is higher than that of the physical mixture (Figure 27a), which is believed to be due to the formation of interparticle voids from the diminished stacking among LDH platelets. Samples from concentrated conditions display narrower pore size distribution and low pore volume than those from dilute conditions (Figure 27).

### 1.2.2. Synthesis and characterisation of ES757@Cuₓ LDH

After the successful large-scale synthesis of ES757@Cu₄ LDH-conc composite with uniform shell morphology and high LDH coverage. The next step is to prepare enough core-shell precursors with different copper loading for the following catalyst study.

Four core-shells were prepared under the same condition as ES757@Cu₄ LDH-conc. The Cu/AI molar ratio in LDH varies from 0.8, 1.3, 2 to 3. The Zn/AI ratio remains at 1. The total amount of metal nitrate remains the same for all syntheses, while the amount for copper, zinc and aluminium nitrate is adjusted according to their molar ratio. Products are named as ES757@Cux LDH, where x is the Cu/AI ratio. The fresh sample undergoes calcination and hydrogen reduction to give a catalyst which is termed as ES757@Cux. The intermediate obtained after the calcination is called ES757@Cux LDO.

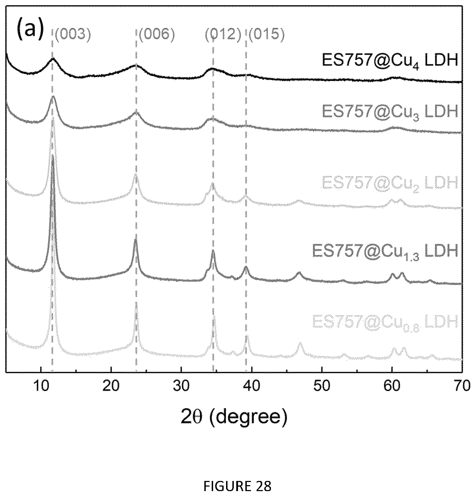

The presence of LDH Bragg reflections in all ES757@Cux LDHs proves the formation of LDH phase and the absence of crystalline impurity (Figure 28).

The composition was determined by the ICP-OES and elemental analysis. The amount of element is normalised to the amount of Al, i.e., the stoichiometry of Al is 1. The actual Cu/AI ratio is close to the expected value (Table 7). Based on the discussion in the previous section, it is known that Al will partially move to silica during the co-precipitation.

A possible chemical formula of the ES757@Cux LDH precursor assuming no Al migration is listed in Table 7.

**Table 7. The expected and experimental Cu: Al molar ratio in ES757@Cuₓ LDHs with their possible chemical formula and impurity.**

| Cu/Al ratio | | Stoichiometry in LDH [CuₐZn_{b}Al_{c}(OH)_{2(a+b+c)}(CO₃)_{c/2}] nH₂O | | | | | | SiOz | Cu₂CO₃ (OH)₂^{b} |
|---|---|---|---|---|---|---|---|---|---|
| Exp^{a} | Real | Cu | Zn | Al | OH | CO₃²⁻ | H₂O | | |
| 0.8 | 0.84 | 0.84 | 1.05 | 1.00 | 5.78 | 0.50 | 1.40 | 3.35 | 0.00 |
| 1.3 | 1.34 | 1.29 | 1.03 | 1.00 | 6.64 | 0.50 | 1.49 | 3.85 | 0.02 |
| 2 | 2.05 | 1.86 | 1.02 | 1.00 | 7.77 | 0.50 | 1.63 | 4.50 | 0.09 |
| 3 | 3.24 | 2.65 | 0.98 | 1.00 | 9.25 | 0.50 | 1.95 | 5.50 | 0.30 |
| 4 | 4.14 | 2.80 | 1.03 | 1.00 | 9.67 | 0.50 | 2.28 | 6.50 | 0.67 |

| | | | | | | | | | |
|---|---|---|---|---|---|---|---|---|---|
| a: expected Cu/Al ratio based on the amount of starting material in co-precipitation. b: Impurity could be a mixture of zinc hydroxide carbonate and copper hydroxide carbonate. | | | | | | | | | |

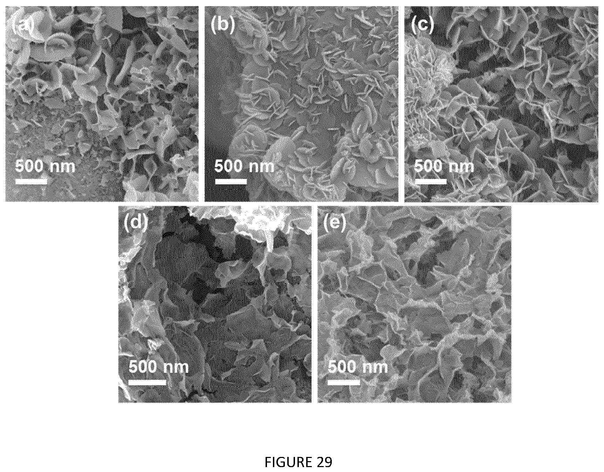

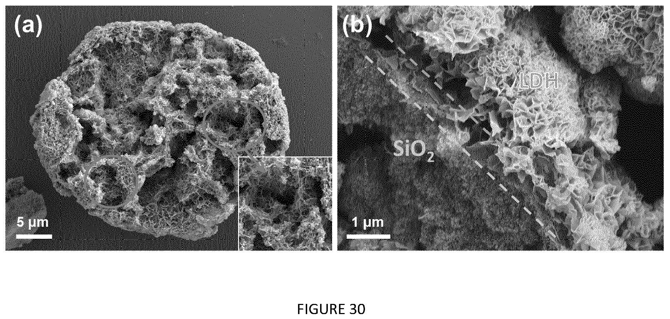

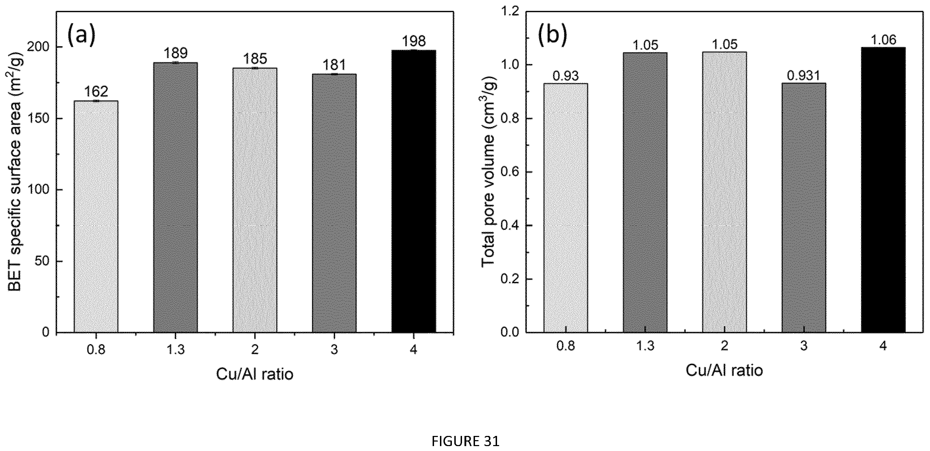

Figures 29 and 30 show HR-SEM images of ES757@Cux LDH. BET surface area and pore volume data are presented in Figure 31.

### 1.3 Synthesis of SiO₂@Cu₂ZnAl LDH with various mesoporous silica

The dispersion of the active site and the mass transfer property of the material are associated with catalystic performance.³⁸ It is assumed that these two properties for catalysts derived from the SiO₂@LDH core-shells are related to the surface area and pore structure of the silica support. To study this, a series of SiO₂@Cu2 LDH composites are prepared using silica with different surface area and pore structure. Four mesoporous silica SBA-15, SBA-16, MCM-41 and MCM-48 were selected. The physical properties of those silica and ES757 are listed in Table 8. SBA-15 and MCM-41 have similar pore structures where 2D round or hexagonal pores are arranged along a hexagonal grid but different surface area.^{39,40} SBA-15 (or MCM-41) has a similar surface area as SBA-16 but a different pore structure. MCM-48 exhibits the highest surface area among all samples. Core-shells were prepared following the exact synthetic condition that is used for ES757@Cu2 LDH. Products are termed after their core, for example, composite containing SBA-15 is called SBA-15@Cu2 LDH.

**Table 8. Physical properties of silicas.**

| Silica | Particle morphology^{a} | Particle size (µm)^{b} | BET specific surface area (m²/g)^{c} | Total pore volume (cm³/g)^{c} | Pore size | Pore structure |
|---|---|---|---|---|---|---|
| ES757 | Spherical | 25 | 273 | 1.63 | 24.2 | Unknown |
| SBA-15 | Wire-like | - | 537 | 0.558 | 1.1, 1.6, 6.8 | 2D round |
| SBA-16 | Spherical | 3.5 | 688 | 0.50 | 1.1,2.1,7 | 3D |
| MCM-41 | Spherical | 1 | 792 | 0.69 | 3.6 | 2D hexagonal |
| MCM-48 | Spherical | 0.604 | 1186 | 0.81 | 3.2 | 3D |

| | | | | | | |
|---|---|---|---|---|---|---|
| a: The particle morphology is checked by the HR-SEM. b: The particle size determined using SEM/TEM. c: The BET specific surface area, total pore volume and pore size is obtained from the N₂ adsorption, and desorption isotherm at 77 K. DFT kernel based on the metal oxide with cylinder pore was used to obtain the pore size. | | | | | | |

ICP-OES and elemental analysis were used to investigate the elemental composition of the SiO₂@Cu2 LDH samples.

**Table 10. The content of elements in the SiO₂@Cu2 LDH revealed by ICP-OES and elemental analysis.**

| Core | Cu | Zn | Al | Si | Na | C | H | N |
|---|---|---|---|---|---|---|---|---|
| SBA-15 | 2.19 | 0.96 | 1.00 | 0.29 | 0.08 | 0.51 | 10.76 | 0.00 |
| SBA-16 | 2.22 | 1.02 | 1.00 | 0.66 | 0.12 | 0.49 | 13.08 | 0.00 |
| MCM-41 | 2.16 | 0.98 | 1.00 | 1.14 | 0.10 | 0.33 | 12.43 | 0.00 |
| MCM-48 | 2.08 | 0.95 | 1.00 | 0.71 | 0.19 | 0.40 | 12.20 | 0.00 |

### 1.4. The effect of Cu loading on ES757@CuₓZnAl LDH derived catalysts

### 1.4.1. Activation and characterisation of ES757@Cuₓ catalyst and relevant LDO intermediates

Calcining CuZnAl LDH at a temperature around or above 600 °C would likely lead to the formation of spinel species, which would be undesirable in this study.⁴¹ However, below this temperature, only dehydroxylation and partial decarboxylation (2^{nd} weight loss stage) of LDH can be achieved. Considering that the 2^{nd} weight loss stage finishes at 300 °C, ES757@Cux LDHs are calcined at 330 °C. The obtained ES757@Cux LDOs are amorphous, as confirmed by XRD. Formation of spinel has been avoided.

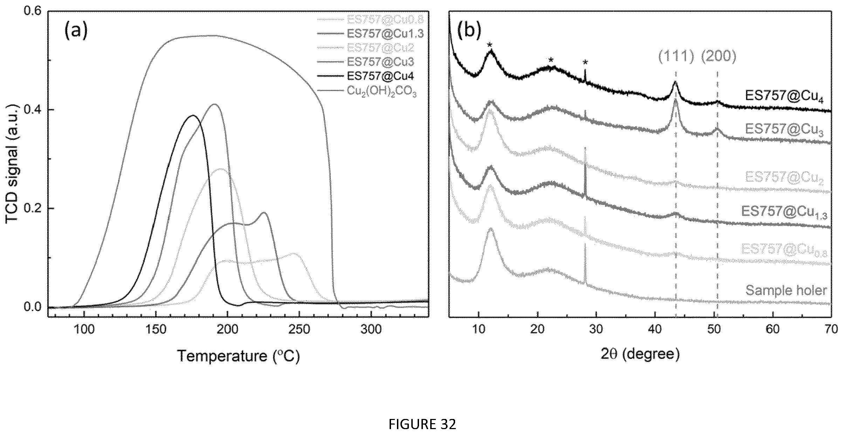

The reducibility of Cu in the ES757@Cux LDO is studied by the H₂-TPR. It is known that ZnO and Al₂O₃ will be reduced by H₂ at temperature above 600 °C and 400 °C, respectively.⁴² Signals recorded between 75-340 °C should correspond to the reduction of Cu moieties (Figure 32a). As expected, the integration of the reduction peak is promoted at the elevated Cu/AI ratio. It is seen that all composites finish their reduction below 280 °C. Bulk CuO will be reduced in hydrogen at 340 °C.⁴³ Well-dispersed CuO species will get reduced at lower temperature.^{35,42,43} It is believed that the LDH framework and core-shell structure facilitate the dispersion of copper.

Based on the H₂-TPD result, the reduction temperature for ES757@Cux LDO is set to 290 °C to ensure a sufficient activation of the catalyst. Figure 32b shows the XRD profiles of catalysts. Cu(111) and Cu(200) reflections are noticed in all samples, the intensity of which increases with raised Cu density. The crystallite domain size of copper is worked out via Rietveld refinement. As expected, high copper loading leads to large copper crystallite (Table 11).

The ICP-OES results signify that the composition of core-shell catalyst is close to expectation (Table 11). A minor amount of carbon and hydrogen are detected which could be due to the adsorption of water and the formation of hydroxide carbonate when the sample was exposed to the air.

**Table 11. The expected Cu/Al ratio, the molar amount of elements in ES757@Cux obtained from ICP (normalised to the amount of Al), the consumed H₂ amount during H₂-TPR measurement and the crystal domain size of copper in the catalysts.**

| Exp^{a} | Cu | Zn | Al | C | H | H₂ (mmol/g) | CDL of Cu (nm)^{b} |
|---|---|---|---|---|---|---|---|
| 0.8 | 0.89 | 1.04 | 1.00 | 0.32 | 1.72 | 2.01 | 1.24 |
| 1.3 | 1.43 | 1.03 | 1.00 | 0.39 | 2.05 | 3.16 | 1.82 |
| 2 | 2.16 | 1.02 | 1.00 | 0.40 | 2.56 | 4.10 | 2.24 |
| 3 | 3.10 | 0.94 | 1.00 | 0.27 | 2.70 | 4.85 | 5.79 |
| 4 | 4.14 | 0.91 | 1.00 | 0.41 | 3.56 | 5.07 | 5.73 |

| | | | | | | | |
|---|---|---|---|---|---|---|---|
| a: the expected Cu/Al ratio based on the amount of copper and aluminium nitrate in the co-precipitation. b: the crystallite domain size is obtained from the Rietveld refinement. | | | | | | | |

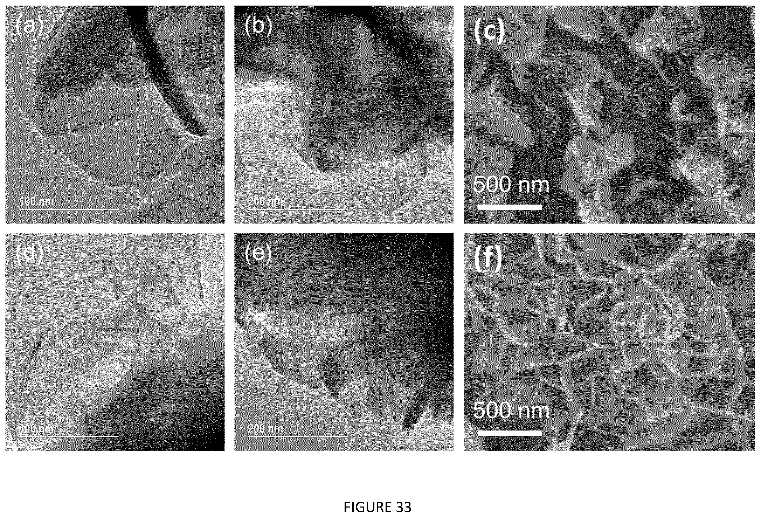

After calcination, LDH was transformed to amorphous layered double oxide with a more porous structure, as revealed by TEM images (Figure 33a, d).

Once the ES757@Cux LDO is reduced to afford the ES757@Cux catalyst, the surface area and total pore volume decrease due to the collapse of the porous oxide structure. Spherical particles, namely copper and zinc oxide nanoparticles, are generated (Figure 33b, e).^{12,35} The core-shell structure remains in catalyst ES757@Cux, and the shell morphology is identical to that of the LDH precursor (Figure 33c,f,).

BET surface area and pore volume data are presented in Figure 32.

### 1.4.2. Catalytic performance and copper dispersion of ES757@Cuₓ

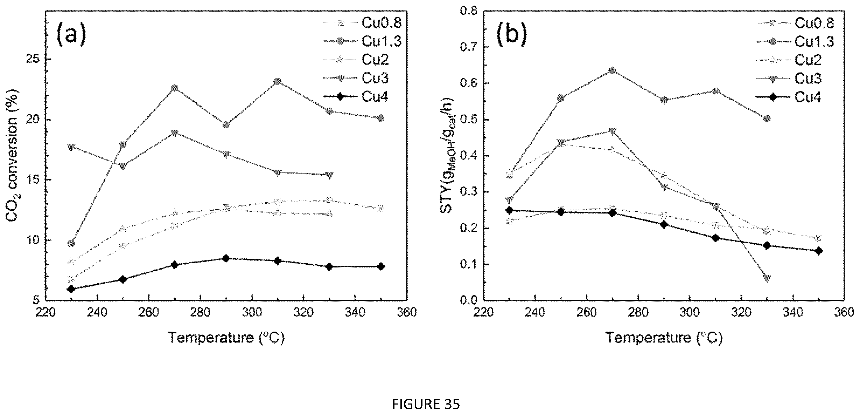

The catalytic performance of ES757@Cux towards MeOH production via CO₂ hydrogenation were evaluated under 45 bar with a mixture of H₂/CO₂ (molar ratio 3:1) at 230-350 °C. The CO₂ conversion is displayed in Figure 35a. The CO₂ conversion of catalysts, except ES757@Cu1.3, show a similar trend within 230-350 °C: increase to the maximum then decrease at further raised temperature. The slope of the change and temperature for the maximum conversion varies depending on the copper density. ES757@Cu3 has a steeper slope compared to the other three samples. ES757@Cu1.3 shows two conversion maxima located at 270 °C and 310 °C, respectively.

ES757@Cu1.3 possesses the highest CO₂ conversion (23%) among all samples. It is noted that the highest transformation value following the order: ES757@Cu1.3 > ES757@Cu3 > ES757@Cu0.8 ≥ ES757@Cu2 > ES757@Cu4.

The space-time yield (STY) of methanol reaches maxima at low temperature, 250 °C or 270 °C. Further raising the reaction temperature results in a lowered methanol yield (Figure 35b). This is mainly due to the lower methanol selectivity at high temperatures. The highest STY (0.64 g_{MeOH}/g_{cat}/h) is obtained by ES757@Cu1.3 at 270 °C.

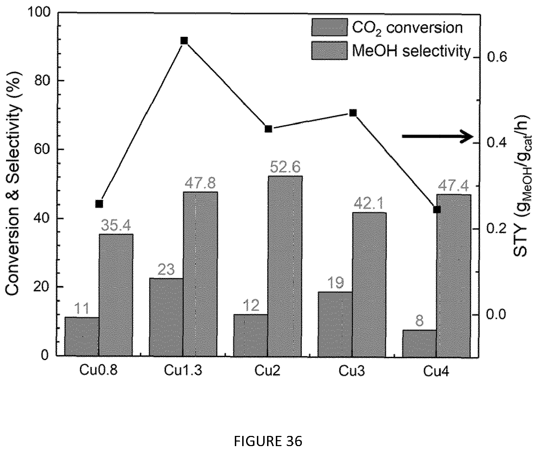

The catalytic performance at 270 °C of ES757@Cux is compared and presented in Figure 36. As the Cu/AI ratio increases, the STY increases to its maximum at Cu/AI =1.3 and then decreases. The trade-off between the CO₂ conversion and methanol selectivity makes ES757@Cu0.8 and ES757@Cu4 has a similar yield. Likewise, the STY of ES757@Cu2 (0.43 g_{MeOH}/g_{cat}/h) and ES757@Cu3 (0.47 g_{MeOH}/g_{cat}/h) are almost identical.

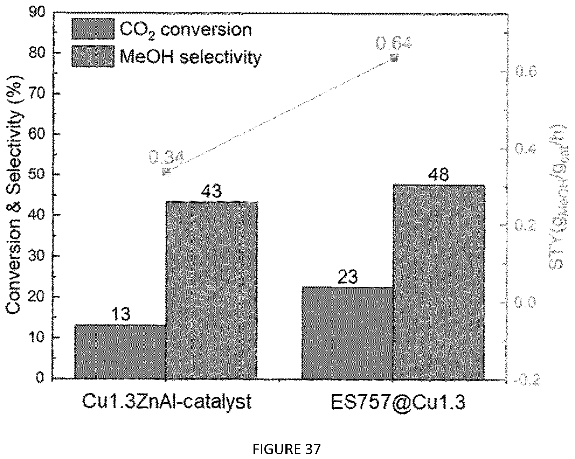

Figure 37 displays the catalytic ability of ES757@Cu1.3 and the catalyst derived from unsupported Cu_{1.3}ZnAl LDH (Cu1.3ZnAl-catalyst). ES757@Cu1.3 exhibits better MeOH yield than Cu1.3ZnAI-catalyst due to its superior CO₂ transform rate. The copper content in core-shell catalyst should be 60% of that in LDH-derived catalyst. It is believed that the core-shell structure promotes LDH dispersion, expose more copper at an even lower copper loading and leads to an excellent catalytic performance.

Reasons behind the varied catalytic ability are investigated. Copper dispersion and copper surface area (S_{cu}) are two factors that determine the catalytic performance of Cu-based catalysts in methanol synthesis.³⁸ N₂O chemisorption accompanied with hydrogen reduction was used to determine copper dispersion and copper surface area. The exposed metallic Cu in the catalyst was firstly oxidized by N₂O then reduced by the hydrogen. The quantity of exposed copper can be calculated from the amount of consumed hydrogen. The dispersion of copper is the fraction of exposed copper among the total copper.⁴⁵ The S_{cu} is the surface area occupied by the metallic copper.^{46,47}

ES757@Cu3 and Cu1.3ZnAI-catalyst were not tested. ES757@Cu1.3 exhibits the largest copper surface area (Table 12), which may explain its highest methanol yield. The trend of methanol STY follows the trend of S_{cu}. Low yield is associated with the low copper surface area.

**Table 12. The Cu loading, crystallite domain length (CDL), Cu dispersion and Cu surface area of ES757@Cux catalysts.**

| Sample | Cu loading (wt %) | CDL of Cu (nm)^{b} | Cu dispersion (%)^{c} | S_{cu} (m²/g_{cat})^{c} | TOF^{d} (mol_{MeOH}/mol_{Cu}/s) |
|---|---|---|---|---|---|
| ES757@Cu0.8 | 15.57 | 1.24 | 17.13 | 17.32 | 0.0052 |
| ES757@Cu1.3 | 19.81 | 1.82 | 25.00 | 32.14 | 0.0071 |
| ES757@Cu2 | 24.98 | 2.24 | 11.13 | 18.05 | 0.0085 |
| ES757@Cu3 | 32.21 | 5.79 | | | |
| ES757@Cu4 | 37.4 | 5.73 | 6.98 | 16.94 | 0.0052 |

| | | | | | |
|---|---|---|---|---|---|
| a: Determined by the ICP-OES. b: Obtained from the Rietveld refinement of the XRD pattern, c: Dispersion and specific surface area of metallic Cu is obtained from N₂O chemisorption results.⁴¹⁻⁴⁷ d: The turnover frequency for methanol production against the surface copper amount. | | | | | |

The TOF of ES757@Cux catalysts are listed in Table 12. ES757@Cux catalysts exhibit a moderate to high TOF value among Cu-based catalysts.^{12,35,44} ES757@Cu2 has the highest TOF at 0.085 mol_{MeOH}/mol_{Cu}/s.

The size of copper nanoparticles is analysed from TEM images (Figure 38). The outstanding TOF value of ES757@Cu2 may be related to its large particle size.

### 1.5 The effect of silica core on catalytic performance

To investigate the effect of silica property, the catalytic performance of catalysts derived from SiO₂@Cu2 LDH (SiO₂ = SBA-15, SBA-16, MCM-41 and MCM-48) were evaluated under 45 bar with a mixture of H₂/CO₂ (molar ratio 3:1) at 230-350 °C. The catalysts were obtained from the calcination and *in-situ* hydrogen reduction of SiO₂@Cu2 LDH precursor and were named as A@Cu2, where A is the name of mother silica.

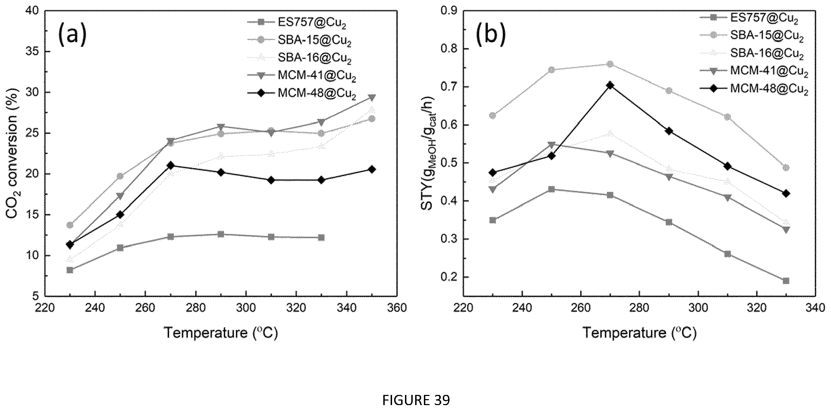

The CO₂ conversion of all catalysts shows a clear increasing trend when the temperature is elevated from 230 °C to about 290 °C (Figure 39a). Variation in the CO₂ conversion and product selectivity leads to the reduced methanol yield at a temperature higher than 290 °C (Figure 39b). The highest methanol yield is obtained by SBA-15@Cu2 at 270 °C and is 0.76 g_{MeOH}/g_{cat}/h.

The CO₂ conversion and methanol selectivity at 270 °C are compared in Figure 40.

The success in using silica with large pores such as SBA-15 rather than the small pore SBA-16, MCM-41 and MCM-48 indicates that a substrate with wide pore shows higher potential in developing an outstanding catalyst that is derived from core@LDH

### 1.6 Catalytic ability comparison with the literature

Previous work reported the catalytic performance of CuZnGa catalyst derived from the exfoliated LDH precursor and an industrial Cu/ZnO/Al₂O₃ catalyst named JM-HiFuel.³⁵ The reaction condition is the same as what is used in this study.

The copper loading in ES757@Cu1.3 is 19.81 wt% and around 24.5wt% in SBA-15@Cu2 as well as MCM-48@Cu2. The copper amount in the CuZnGa and HiFuel is 33.5wt% and 50wt%, respectively. It is noticed that ES757@Cu1.3, SBA-15@Cu2 and MCM-48@Cu2 have superior catalytic performance than both the CuZnGa and the industrial catalyst at an even lower copper loading (Figure 41).

While specific embodiments of the invention have been described herein for the purpose of reference and illustration, various modifications will be apparent to a person skilled in the art without departing from the scope of the invention as defined by the appended claims.

### REFERENCES

1. Yu, K. M. Curcic, I. Gabriel, J. & Tsang, S. C. E. ChemSusChem. 1, 893-899 (2008).
2. Turner, J. et al. Int. J. Energy Res. 32, 379-407 (2008).
3. Song, C. Catal. Today. 115, 2-32 (2006).
4. Fujitani, T. Nakamura, J. Appl. Catal. A. 191, 111-129 (2000).
5. Liao, F. Zeng, Z. Eley, C. Lu, Q. Hong, X. & Tsang, S. C. E. Angew. Chem. Int. Ed. 51, 5832-5836 (2012).
6. Zander, S. et al. Angew. Chem. Int. Ed. 52, 6536-6540 (2013).
7. Spencer, M.S. Top. Catal. 8, 259-266 (1999).
8. Fujitani, T. & Nakamura, J. Catal. Lett. 56, 119-124 (1998).
9. Kanai, Y. et al. Catal. Lett. 27, 67-78 (1994).
10. Fujita, S. Usui, M. Ito, H. Takezawa, N. J. Catal. 157, 403-413 (1995).
11. Choi, Y. Futagami, K. Fujitani, T. Nakamura, J. Appl. Catal. A. 208, 163-167 (2001).
12. Behrens, M. et al. Science. 336, 893-897 (2012).
13. Arena, F. Barbera, K. Italiano, G. Bonura, G. & Spadaro, L. J. Catal. 249, 185-194 (2007).
14. Saito, M. Fujitani, T. Takeuchi, M. & Watanabe, T. Appl. Catal. A. 138, 311-318 (1996).
15. Kurtz, M. Wilmer, H. Genger, T. Hinrichsen, O. & Muhler, M. Catal. Lett. 86, 77-80 (2003).
16. An, X. et al. Catal. Lett. 118, 264-269 (2007).
17. Weigel, J. Koeppel, R. A. Baiker, A. & Wokaun, A. Langmuir. 12, 5319-5329 (1996).
18. Yu, K. M. et al. Nat. Commun. 3, 1230 (2012).
19. Tong, W. Cheung, K. West, A. Yu, K. M. & Tsang, S. C. E. Phys. Chem. Chem. Phys. 15, 7240-7248 (2013).
20. Tong, W. West, A. Cheung, K. Yu, K. M. & Tsang, S. C. E. ACS catal. 3, 1231-1244 (2013
21. F. Cavani, F. Trifirò and A. Vaccari, Catal. Today, 1991, 11, 173-301.
22. L. B. Staal, S. S. Charan Pushparaj, C. Forano, V. Prevot, D. B. Ravnsbaek, M. Bjerring and U. G. Nielsen, J. Mater. Chem. A, 2017, 5, 21795-21806.
23. S. S. C. Pushparaj, C. Forano, V. Prevot, A. S. Lipton, G. J. Rees, J. V. Hanna and U. G. Nielsen, J. Phys. Chem. C, 2015, 119, 27695-27707.
24. S. Ramadan, T. W. Hambley, B. J. Kennedy and P. A. Lay, Inorg. Chem., 2004, 43, 2943-2946.
25. A. J. Pell, G. Pintacuda and C. P. Grey, Prog. Nucl. Magn. Reson. Spectrosc., 2019, 111, 1-271.
26. S. K. Matam, A. J. O'Malley, C. R. A. Catlow, Suwardiyanto, P. Collier, A. P. Hawkins, A. Zachariou, D. Lennon, I. Silverwood, S. F. Parker and R. F. Howe, Catal. Sci. Technol., 2018, 8, 3304-3312.
27. A. A. Romero, M. D. Alba and J. Klinowski, J. Phys. Chem. B, 1998, 102, 123-128.
28. N. D. Jensen, N. T. Duong, R. Bolanz, Y. Nishiyama, C. A. Rasmussen, J. Gottlicher, R. Steininger, V. Prevot and U. G. Nielsen, Inorg. Chem., 2019, 58, 6114-6122.
29. K. Chen, M. Abdolrhamani, E. Sheets, J. Freeman, G. Ward and J. L. White, J. Am. Chem. Soc., 2017, 139, 18698-18704.
30. M. Thommes, K. Kaneko, A. V. Neimark, J. P. Olivier, F. Rodriguez-Reinoso, J. Rouquerol and K. S. W. Sing, Pure Appl. Chem., 2015, 87, 1051-1069.
31. J. B. Condon, in Surface Area and Porosity Determinations by Physisorption, 2006, pp. 1-27.
32. C. Ye, M. Q. Wang, S. J. Bao and C. Ye, ACS Appl. Mater. Interfaces, 2019, 11, 30887-30893.
33. C. Chen, A. Wangriya, J. C. Buffet and D. O'Hare, Dalt. Trans., 2015, 44, 16392-16398.
34. F. J. Sotomayor, K. A. Cychosz and M. Thommes, Acc. Mater. Surf. Res, 2018, 3, 34-50.
35. M. M. J. Li, C. Chen, T. Ayvall, H. Suo, J. Zheng, I. F. Teixeira, L. Ye, H. Zou, D. O'Hare and S. C. E. Tsang, ACS Catal., 2018, 8, 4390-4401.
36. Robert Strachan, University of Oxford, 2019.
37. W. L. J. Kwok, University of Oxford, 2020.
38. X. Jiang, X. Nie, X. Guo, C. Song and J. G. Chen, Chem. Rev., 2020, 120, 7984-8034.
39. M. Martinez-Carmona, Y. K. Gun'ko and M. Vallet-Regí, Pharmaceutics, 2018, 10, 1-29.
40. L. Angiolini, B. Cohen and A. Douhal, Int. J. Mol. Sci., 2019, 20, 9-12.
41. P. Kowalik, M. Konkol, M. Kondracka, W. Próchniak, R. Bicki and P. Wiercioch, Appl. Catal. A Gen., 2013, 464-465, 339-347.
42. Š. Hajduk, V. D. B. C. Dasireddy, B. Likozar, G. Dražić and Z. C. Orel, *Appl. Catal. 8 Environ.,* 2017, **211**, 57-67.
43. M. Turco, G. Bagnasco, C. Cammarano, P. Senese, U. Costantino and M. Sisani, Appl. Catal. 8 Environ., 2007, 77, 46-57.
44. P. Gao, L. Zhong, L. Zhang, H. Wang, N. Zhao, W. Wei and Y. Sun, Catal. Sci. Technol., 2015, 5, 4365-4377.
45. G. V. Sagar, P. V. R. Rao, C. S. Srikanth and K. V. R. Chary, J. Phys. Chem. B, 2006, 110, 13881-13888.
46. D. S. Brands, E. K. Poels, T. A. Krieger, O. V. Makarova, C. Weber, S. Veer and A. Bliek, Catal. Letters, 1996, 36, 175-181.
47. W. R. A. M. Robinson and J. C. Mol, Appl. Catal., 1990, 63, 165-179.

## Claims

1. A particle having a silica core and a layered double hydroxide shell, wherein the layered double hydroxide comprises Cu²⁺ and Zn²⁺.

2. The particle of claim 1, wherein the combined amounts of Cu²⁺ and Zn²⁺ accounts for at least 85 mol% of all monovalent and divalent metal cations present within the layered double hydroxide, and wherein one or more of Mg²⁺, Ni²⁺ and Ca²⁺ form the optional balance of all monovalent and divalent metal cations within the layered double hydroxide.

3. The particle of claim 1 or 2, wherein one or more of Al³⁺, Ga³⁺, In³⁺ and Zr⁴⁺ accounts for all trivalent and tetravalent metal cations present within the layered double hydroxide.

4. The particle of claim 1, 2 or 3, wherein any one or more of the following statements (A) to (D) applies:
(A) the molar ratio of Cu²⁺ to all trivalent and tetravalent metal cations present within the layered double hydroxide is 0.8 - 4.0;
(B) the molar ratio of Cu²⁺ to Zn²⁺ within the layered double hydroxide is 0.8 - 4.5;
(C) the molar ratio of Zn²⁺ to all trivalent and tetravalent metal cations present within the layered double hydroxide is 0.7 - 1.3;
(D) one or more of an inorganic oxyanion, a halide and hydroxide accounts for all anions present within the layered double hydroxide interlayer.

5. The particle of claim 1, wherein the layered double hydroxide comprising Cu²⁺ and Zn²⁺ is of formula (I):
[(Cu²⁺ₓZn²⁺_{y}M²⁺_{z})_{1-*q*}M³⁺*_{q}*(OH)₂]^{*q*+}(X^{*n*-})_{*q*/}*ₙ·w*(H₂O)·*u*(OS) (I)
wherein
M²⁺ is at least one divalent metal cation;
M³⁺ is at least one trivalent metal cation;
0.1≤ (*x*+*y*) ≤1;
*z* = 1 - (*x*+*y*);
0<*q*<0.9;
0≤*w*≤10;
0≤*u*≤10
X is an anion;
OS is an organic solvent capable of hydrogen bonding to water; and
*n* is the charge on anion X.

6. The particle of any one of the preceding claims, wherein either one or both of the following statements (A) and (B) applies:
(A) the silica core accounts for 10 - 50 wt% of the particle and the layered double hydroxide accounts for 50 - 90 wt% of the particle;
(B) the silica core has an average particle size of 0.1 - 500 µm.

7. A catalyst precursor, wherein the catalyst precursor is a thermally treated form of the particle as defined in any one of claims 1 to 6.

8. A catalyst precursor in the form of particle having a silica core and a layered double oxide shell, wherein the layered double oxide comprises Cu²⁺ and Zn²⁺.

9. A catalyst, wherein the catalyst is a reduced or partially reduced form of the catalyst precursor of claim 7 or 8.

10. A catalyst in the form of a particle having a silica core and a shell formed from Al₂O₃ and ZnO, wherein the shell comprises either or both of free Cu and a free Cu-Zn alloy.

11. The catalyst of claim 9 or 10, wherein either one or both of the following statements (A) and (B) applies:
(A) the molar ratio of Cu to Zn in the catalyst is 0.8 - 4.5;
(B) the molar ratio of Cu to Al in the catalyst is 0.8 - 4.0.

12. The catalyst of claim 9, 10 or 11, wherein the free Cu and the free Cu-Zn alloy are in the form of particles having an average size of 1 - 30 nm.

13. The catalyst of any one of claims 9 to 12, wherein any one or more of the following statements (A) to (C) applies:
(A) the catalyst comprises 10 - 45 wt.% of Cu;
(B) the catalyst comprises 5 - 25 wt.% of Zn;
(C) the catalyst comprises 1-12 wt.% of Al.

14. A process for the preparation of methanol, the process comprising the step of:
a) contacting a catalyst as defined in any one of claims 9 to 13 with a mixture of hydrogen and carbon dioxide.

15. The process of claim 14, wherein step a) is conducted at a temperature of 200-380°C or 240-320°C.
